(19) Europäisches Patentamt
European Patent Office
Office européen des brevets

(11) Veröffentlichungsnummer **0 001 054**
**A2**

(12) # EUROPÄISCHE PATENTANMELDUNG

(21) Anmeldenummer: 78100637.4

(22) Anmeldetag: 09.08.78

(51) Int. Cl.$^2$: C 07 D 211/90
C 07 D 401/14, C 07 D 405/14
C 07 D 409/14, C 07 D 471/04
A 61 K 31/44
(C07D471/04,239/00,221/00)

(30) Priorität: 24.08.77 DE 2738153

(43) Veröffentlichungstag der Anmeldung: 21.03.79 Patentblatt 79/6

(84) Benannte Vertragsstaaten: BE CH DE FR GB NL

(71) Anmelder: Bayer Aktiengesellschaft
Zentralbereich Patente,Marken und Lizenzen Bayerwerk
D-5090 Leverkusen 1(DE)

(72) Erfinder: Bossert, Friedrich
Claudiusweg 7
D-5600 Wuppertal 1(DE)

(72) Erfinder: Heise, Arend
Möbeck 50
D-5600 Wuppertal 1(DE)

(72) Erfinder: Kazda, Stanislav
Pahlkestrasse 55 N
D-5600 Wuppertal 1(DE)

(72) Erfinder: Klauke, Erich
Eichendorffweg 8
D-5068 Odenthal(DE)

(72) Erfinder: Meyer, Horst
Theodor-Heuss-Strasse 110
D-5600 Wuppertal-1(DE)

(72) Erfinder: Stoepel, Kurt
In den Birken 69
D-5600 Wuppertal-1(DE)

(72) Erfinder: Towart, Robertson
Claudiusweg 9
D-5600 Wuppertal-1(DE)

(72) Erfinder: Wehinger, Egbert
Donnenbergerstrasse 90
D-5620 Velbert 15(DE)

(54) **2-Pyridyl-1,4-dihydropyridine, Verfahren zu ihrer Herstellung sowie ihre Verwendung als Arzneimittel.**

(57) Die vorliegende Erfindung betrifft neue 2-Pyridyl-1,4-dihydropyridine, mehrere Verfahren zu ihrer Herstellung sowie ihre Verwendung als Arzneimittel, vorzugsweise als gefäßbeeinflussende Mittel, insbesondere als Coronarmittel.

EP 0 001 054 A2

Croydon Printing Company Ltd

0001054



BAYER AKTIENGESELLSCHAFT 5090 Leverkusen, Bayerwerk
Zentralbereich KS/bc
Patente, Marken und Lizenzen Ib (Pha)

# 2-Pyridyl-1,4-dihydropyridine, Verfahren zu ihrer Herstellung sowie ihre Verwendung als Arzneimittel

Die vorliegende Erfindung betrifft neue 2-Pyridyl-1,4-dihydropyridine, mehrere Verfahren zu ihrer Herstellung sowie ihre Verwendung als Arzneimittel, vorzugsweise als gefäßbeeinflussende Mittel, insbesondere als Coronarmittel.

Es ist bereits bekannt geworden, daß bestimmte 1,4-Dihydropyridine interessante pharmakologische Eigenschaften besitzen (F. Bossert und W. Vater, Die Naturwissenschaften (1971), 58. Jahrgang, Heft 11, Seite 578).

Die vorliegende Erfindung betrifft neue 2-Pyridyl-1,4-dihydropyridine der allgemeinen Formel

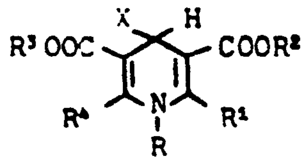 (I)



BAD ORIGINAL

in welcher

R für Wasserstoff, gegebenenfalls substituierten Alkyl oder Aralkyl steht,

$R^1$ für einen gegebenenfalls substituierten α-, β- oder γ-Pyridyl-Reste steht,

$R^2$ und $R^3$ gleich oder verschieden sind und jeweils für einen gegebenenfalls substituierten Alkyl-, Alkenyl- oder Alkinyl-Rest stehen, welche gegebenenfalls in der Kette durch Heteroatome wie Sauerstoff, Schwefel oder eine -NR'-Gruppe unterbrochen sind, wobei R' Wasserstoff, Alkyl oder Aralkyl bedeutet, wobei die vorgenannten Reste, geradkettig, verzweigt oder cyclisch vorliegen,

$R^4$ für Wasserstoff, gegebenenfalls substituiertes Alkyl, [illegible]

X für gegebenenfalls substituiertes Alkyl oder für einen Arylrest steht, der gegebenenfalls ein, zwei oder drei Substituenten aus der Gruppe Nitro, Cyano, [illegible], Trifluormethyl, Trifluormeth[illegible], Hydroxy, Amino, Alkyl, Alkoxy, Alkoxycarbonyl, Acyloxy, Acylamino, Monoalkylamino, Dialkylamino und $S(O)_m$-Alkyl trägt, wobei m eine Zahl von 0 bis 2 bedeutet und die vorgenannten Substituenten gleich oder verschieden sind

oder

für gegebenenfalls substituiertes Aralkyl, Styryl, Cycloalkyl, Cycloalkenyl, Chinolyl, Isochinolyl, Pyridyl, Pyrimidyl, Furyl, Thenyl oder Pyryl steht,

oder

$R^3$ und $R^4$ gemeinsam für einen Alkylen- oder Alkenylenrest stehen, der gegebenenfalls durch Heteroatome wie



BAD ORIGINAL

Sauerstoff, Schwefel oder Stickstoff unterbrochen ist,

sowie ihre pharmakologisch unbedenklichen Salze.

Es wurde gefunden, daß man 2-Pyridyl-1,4-dihydropyridine der Formel (I) erhält, wenn man

a) einen ß-Ketocarbonsäureester der Formel

$$R^1\text{-CO-CH}_2\text{-COOR}^2 \qquad \text{(II)}$$

in welcher

$R^1$ und $R^2$ die oben angegebene Bedeutung besitzen,

mit einem Amin oder einem Salz des Amins der Formel

$$H_2N\text{-R} \qquad \text{(III)}$$

in welcher

R die oben angegebene Bedeutung hat,

gegebenenfalls nach Isolierung des aus (II) und (III) entstehenden Enamins der Formel

NH-R

$R^1$-C=CH-$COOR^2$ (IV)

in welcher

R, $R^1$ und $R^2$ die oben angegebene Bedeutung besitzen,

mit einem Yliden-Derivat der Formel

O

X-CH=C-C-$R^4$ (V)

$COOR^3$

in welcher

X, $R^3$ und $R^4$ die oben angegebene Bedeutung besitzen,

umsetzt,



BAD ORIGINAL

b) oder einen ß-Ketocarbonsäureester der Formel

$$R^4\text{-CO-CH}_2\text{-COOR}^3 \qquad \text{(VI)}$$

in welcher
$R^3$ und $R^4$ die oben angegebene Bedeutung besitzen, mit einem Amin oder einem Salz des Amins der Formel

$$H_2N\text{-R} \qquad \text{(III)}$$

in welcher
R die oben angegebene Bedeutung hat,
gegebenenfalls nach Isolierung des aus (IV) und (III) entstehenden Enamins der Formel

$$R^4\text{-}\underset{}{\overset{\text{NH-R}}{\text{C}}}\text{=CH-COOR}^3 \qquad \text{(VII)}$$

in welcher
R, $R^3$ und $R^4$ die oben angegebene Bedeutung besitzen, mit einem Yliden-Derivaten der Formel

$$\text{X-CH=}\underset{\text{COOR}^2}{\text{C}}\text{-CO-R}^1 \qquad \text{(VIII)}$$

in welcher
$R^1$, $R^2$ und X die oben angegebene Bedeutung besitzen, umsetzt,

c) oder einen ß-Ketocarbonsäureester der Formel

$$R^4\text{-CO-CH}_2\text{-COOR}^3 \qquad \text{(VI)}$$

in welcher
$R^3$ und $R^4$ die oben angegebene Bedeutung besitzen, mit einem Enamin der Formel

BAD ORIGINAL

$$R^1-\overset{NH-R}{C}=CH-COOR^2 \quad (IV)$$

in welcher
R, $R^1$ und $R^2$ die oben angegebene Bedeutung besitzen,

und mit einem Aldehyd der Formel

$$X-CHO \quad (IX)$$

in welcher
X die oben angegebene Bedeutung hat,

umsetzt,

d. oder einen ß-Ketocarbonsäureester der Formel

$$R^1-CO-CH_2-COOR^2 \quad (II)$$

in welcher
$R^1$ und $R^2$ die oben angegebene Bedeutung besitzen,

mit einem Enamin der Formel

$$R^4-\overset{NH-R}{C}=CH-COOR^3 \quad (VII)$$

in welcher
R, $R^3$ und $R^4$ die oben angegebene Bedeutung besitzen,

und mit einem Aldehyd der Formel

$$X-CHO \quad (IX)$$

in welcher
X die oben angegebene Bedeutung hat,

unsetzt und gegebenenfalls aus den gemäß der Verfahrensvarianten a) bis d) erhaltenen Verbindungen in üblicher Weise ein Salz herstellt.

Überraschenderweise zeigen die erfindungsgemäßen 2-Pyridyl-1,4-dihydropyridine eine sehr starke gefäßbeeinflussende, insbesondere Coronarwirkung. Die erfindungsgemäßen Stoffe stellen somit eine Bereicherung der Pharmazie dar.

A) Verwendet man 3,4,5-Trimethoxybenzyliden-acetessigsäuremethylester, α-Picolinoyl-essigsäuremethylester und Methylamin (bzw. ß-Methylamino-ß-(α-pyridyl)-acrylsäuremethylester als Ausgangsstoffe, so kann der Reaktionsablauf für Variante a) durch folgendes Formelschema wiedergegeben werden:

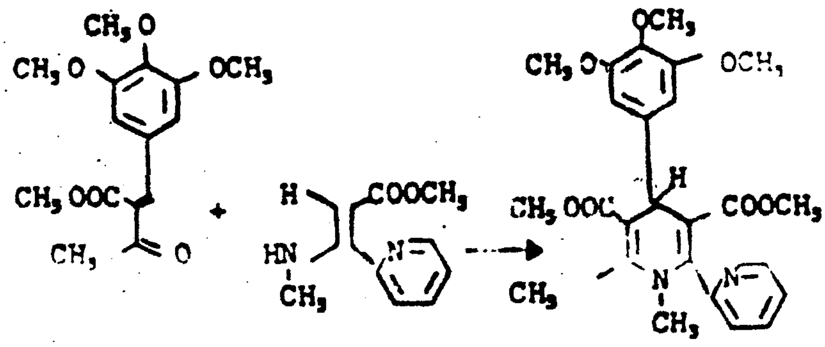

(a)

B) Verwendet man 3-Chlorbenzyliden-γ-pyridoylessigsäureäthylester, γ-Methoxyacetessigsäureäthylester und Ammoniak als Ausgangskomponenten, so kann der Reaktionsablauf für Variante b) durch das folgende Formelschema wiedergegeben werden:

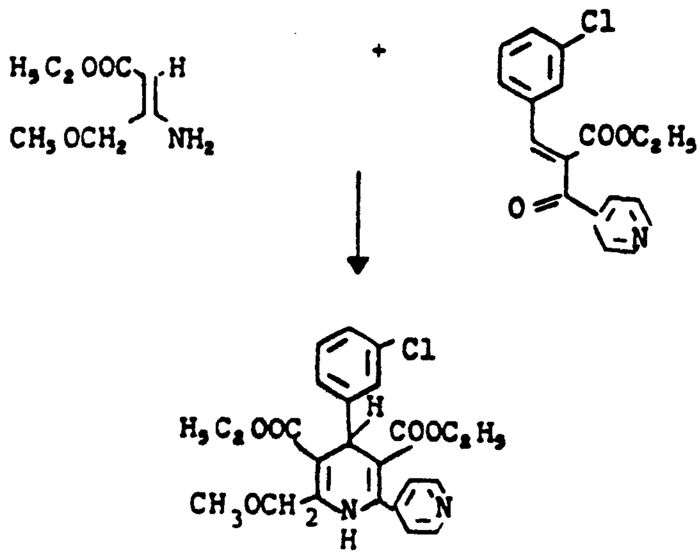

(b)

C) Verwendet man 3-Aethyl-sulfonylbenzaldehyd, β-(β'-Pyridyl)-β-amino-acrylsäurepropylester und Propionylessigsäureäthylester als Ausgangskomponenten, so kann der Reaktionsablauf für Variante c) durch das folgende Formelschema wiedergegeben werden :

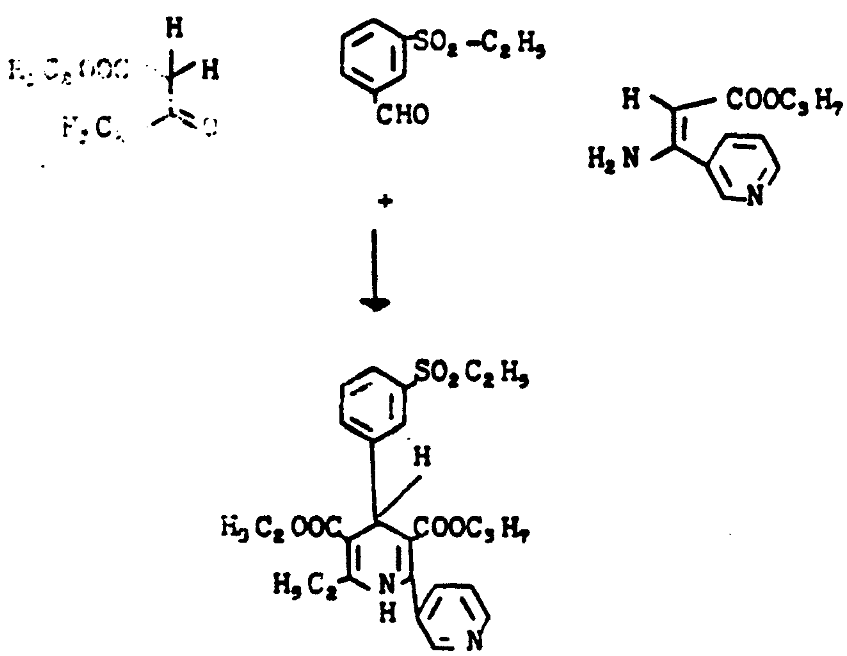

(c)

D) Verwendet man Pyridin-2-aldehyd, α-Pyridoyl-essigsäure-butylester und β-Amino-γ-äthoxy-crotonsäure-äthylester als Ausgangsstoffe, so kann der Reaktionsablauf für Variante d) durch das folgende Formelschema wiedergegeben werden :

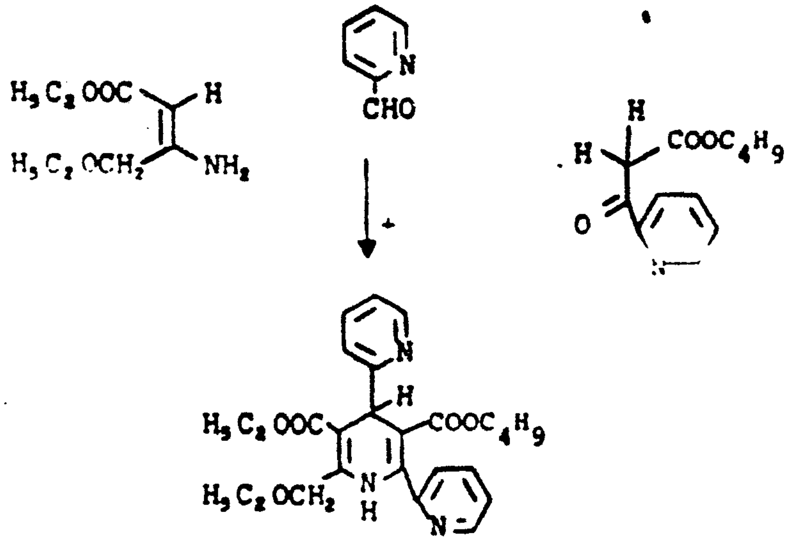

(d)

Von besonderem Interesse sind erfindungsgemäße Verbindungen der Formel (I), in welcher

R für Wasserstoff, Alkyl mit 1 bis 4 Kohlenstoffatomen, Benzyl oder Phenäthyl steht,

$R^1$ für einen α-, β- oder γ-Pyridyl-Rest steht,

$R^2$ für einen geradkettigen oder verzweigten Alkylrest mit 1 bis 6 Kohlenstoffatomen, insbesondere mit 1 bis 4 Kohlenstoffatomen, der gegebenenfalls durch ein Sauerstoffatom unterbrochen ist steht oder für einen Alkenyl- oder Alkinyl-Rest mit bis zu 4 Kohlenstoffatomen steht,

$R^3$ für einen geradkettigen, verzweigten oder cyclischen Alkyl- oder Alkenyl-Rest, der gegebenenfalls substituiert ist und durch ein Sauerstoffatom in der Kette unterbrochen ist, steht, oder für einen Alkinylrest mit bis zu 4 Kohlenstoffatomen steht,

$R^4$ für Wasserstoff oder Alkyl mit 1 bis 4 Kohlenstoffatomen steht und

X für einen Phenyl- oder Naphthyl-Rest steht, der gegebenenfalls durch 1, 2 oder 3 Substituenten aus der Gruppe Nitro, Halogen, Trifluormethyl, Cyano, Amino, Monoalkylamino, Dialkylamino, Alkyl, Alkoxy, Alkylmercapto, Alkylsulfinyl, Alkylsulfonyl, Azido [illegible]alkoxy substituiert ist, wobei die genannten Alkyl- und Alkoxyreste vorzugsweise 1 bis 4, insbesondere 1 oder 2 Kohlenstoffatome enthalten,
oder für gegebenenfalls substituiertes Benzyl, Phenäthyl, Styryl, Cycloalkyl, Cycloalkenyl mit 5 bis 7 Kohlenstoffatomen, Chinolyl, Isochinolyl, Pyridyl, Pyrimedyl, Furyl, Thienyl oder Pyrylrest steht, wobei als Substituenten vorzugsweise Alkyl, Alkoxy, Dialkylamino, mit jeweils 1 bis 4 Kohlenstoffatomen, insbesondere 1 oder 2 Kohlenstoffatome, Halogen oder Nitro in Frage kommen.

Falls nicht ausdrücklich anders angegeben bedeutet der Ausdruck Alkyl in der vorliegenden Anmeldung geradkettiges, verzweigtes oder cyclisches Alkyl mit 1 bis 8 Kohlenstoffatomen, insbesondere mit 1 bis 6 Kohlenstoffatomen. Beispielhaft seien genannt Methyl, Äthyl, Propyl, Isopropyl, n-Butyl, iso-Butyl,

BAD ORIGINAL

tert.-Butyl, n-Pentyl, iso-Pentyl, n-Hexyl, Cyclohexyl, Cycloheptyl, n-Heptyl, n-Octyl, iso-Octyl, Cyclopropyl und Cyclobutyl.

Der Ausdruck Alkenyl steht vorzugsweise für geradkettiges, verzweigtes oder cyclisches Alkenyl mit 2 bis 6, insbesondere 2 bis 4, Kohlenstoffatomen. Beispielhaft seien genannt Propenyl-(2), Butenyl-(3), Pentenyl-(2) und Cyclohexenyl.

Der Ausdruck Alkinyl steht vorzugsweise für geradkettiges oder verzweigtes Alkinyl mit bis zu 6 Kohlenstoffatomen. Beispielhaft seien genannt Propinyl-(2) und Butinyl-(3).

Die vorgenannten Alkyl-, Alkenyl- und Alkinylreste sind gegebenenfalls durch Sauerstoffatome oder Stickstoffatome in der Kette unterbrochen, insbesondere durch ein Sauerstoffatom.

Halogen steht vorzugsweise für Fluor, Chlor, Brom oder Jod, insbesondere für Fluor oder Chlor.

Der Arylrest des Substituenten X kann vorzugsweise 1 bis 3, insbesondere 1 oder 2 gleiche oder verschiedene Substituenten tragen. Als Substituenten seien aufgeführt: Phenyl, Alkyl mit vorzugsweise 1 bis 4, insbesondere 1 oder 2 Kohlenstoffatomen, wie Methyl, Aethyl, n- und i-Propyl und n-, i- und t-Butyl; Alkoxy mit vorzugsweise 1 bis 4, insbesondere 1 oder 2 Kohlenstoffatomen, wie Methoxy, Aethoxy, n- und i-Propyl- und n-, i- und t-Butyloxy; Trifluormethyl; Trifluormethoxy; Hydroxy;



BAD ORIGINAL

Halogen, vorzugsweise Fluor, Chlor, Brom und Jod, insbesondere Chlor und Brom; Cyano; Nitro; Azido; Amino; Monoalkyl- und Dialkylamino mit vorzugsweise 1 bis 4, insbesondere 1 oder 2 Kohlenstoffatomen je Alkylgruppe, wie Methylamino, Methyläthylamino, n- und i-Propylamino und Methyl-n-butylamino; Carbalkoxy mit vorzugsweise 2 bis 4, insbesondere 2 oder 3 Kohlenstoffatomen, wie Carbomethoxy und Carboäthoxy; Acylamino mit vorzugswiese 1 bis 4, insbesondere 2 oder 3 Kohlenstoffatomen, wie Acetylamino und Propionylamino; Acyloxy mit vorzugsweise 2 bis 6, insbesondere 2 bis 4 Kohlenstoffatomen wie Acetyloxy und Propionyloxy; $S(O)_m$-Alkyl, worin m eine Zahl von 0 bis 2, insbesondere 0 oder 2 bedeutet und Alkyl vorzugsweise 1 bis 4, insbesondere 1 oder 2 Kohlenstoffatome enthält, wie Methylthio, Aethylthio, Methylsulfoxyl, Aethylsulfoxyl, Methylsulfonyl und Aethylsulfonyl.

Cycloalkyl bedeutet vorzugsweise mono-, bi- und tricyclisches Cycloalkyl mit vorzugsweise 3 bis 10, insbesondere 3, 5 oder 6 Kohlenstoffatomen. Beispielhaft seien genannt : Cyclopropyl, Cyclobutyl, Cyclopentyl, Cyclohexyl, Cycloheptyl, Bicyclo-/2,2,1/-heptyl, Bicyclo-/2,2,2/-octyl und Adamantyl.

Cycloalkenyl bedeutet vorzugsweise mono-, bi- und tricyclisches Cycloalkenyl mit vorzugsweise 5 bis 10, insbesondere 5, 6 oder 7 Kohlenstoffatomen. Beispielhaft seien Cyclopentenyl, Cyclohexenyl und Cycloheptenyl genannt.

Alkyl und Alkoxy als Substituenten im Naphthyl-, Chinolyl-, Isochinolyl-, Pyridyl-, Pyrimidyl-, Diphenyl-, Furyl- oder Pyrrylrest X bedeuten geradkettiges oder verzweigtes Alkyl



BAD ORIGINAL

und Alkoxy mit vorzugsweise 1 bis 6, insbesondere 1 bis 4 Kohlenstoffatomen. Beispielhaft seien Methyl, Aethyl, n- und i-Propyl, n-, i- und t-Butyl sowie Methoxy, Aethoxy, n- und i-Propoxy und n-, i- und t-Butoxy genannt.

Halogen als Substituent des Naphthyl-, Chinolyl-, Isochinolyl- Pyridyl-, Pyrimidyl-, Diphenyl-, Furyl- und Pyrrylrestes X bedeutet Fluor, Chlor, Brom und Jod, insbesondere Fluor und Chlor.

Dialkylamino als Substituent des Naphthyl-, Chinolyl, Isochinolyl-, Pyridyl-, Pyrimidyl-, Diphenyl-, Furyl- oder Pyrrylrestes X enthält vorzugsweise 1 bis 4, insbesondere 1 oder 2 Kohlenstoffatome je Alkylgruppe. Als Alkylgruppen seien beispielhaft genannt : Methyl, Aethyl, n- und i-Propyl und n-, i- und t-Butyl.

Salze der Verbindungen der Formel I sind alle nichttoxischen, physiologisch verträglichen Säureadditionssalze. Als anorganische und organische Säuren, die mit den Verbindungen der Formel I solche Salze bilden, seien beispielhaft genannt : Halogenwasserstoffsäuren, z.B. Chlor- und Bromwasserstoffsäure, insbesonders Chlorwasserstoffsäure, Phosphorsäuren, Schwefelsäuren, Salpetersäure, mono- und bifunktionelle Carbonsäuren und Hydroxycarbonsäuren, z.B.Essigsäure, Maleinsäure, Bernsteinsäure, Fumarsäure, Weinsäure, Zitronensäure, Salicylsäure, Sorbinsäure, Milchsäure und 1,5-Naphthalincarbonsäure.

Die Salze werden nach allgemein üblichen Methoden, z.B. durch Auflösen der Base in Aether und Versetzen der Lösung mit der betreffenden Säure hergestellt.

Die erfindungsgemäß verwendbaren Pyridoylessigsäureester der Formel II sind bekannt oder können nach bekannten Verfahren hergestellt werden (vgl. A.Pinner B 34.4234.-53 (1901)).

Als Beispiele seien genannt :

| | | | | |
|---|---|---|---|---|
| α-, | -β- | oder -γ-Pyridoylessigsäuremethylester | | |
| " | " | " " | " | äthylester |
| " | " | " " | " | propylester |
| " | " | " " | " | isopropylester |
| " | " | " " | " | butylester |
| " | " | " " | " | isobutylester |
| " | " | " " | " | tert.-butylester |

Die erfindungsgemäß verwendbaren Amine der Formel III sind bereits bekannt.

Als Beispiele seien genannt:
Ammoniak, Methylamin, Aethylamin, Propylamin, Butylamin, Isopropylamin, Isobutylamin, Allylamin , Benzylamin.

Die erfindungsgemäß verwenbaren Enamin-β-keto-carbonyl-Verbindungen der Formel IV können nach allgemein bekannten Methoden aus den entsprechenden β-Diketo-Verbindung hergestellt werden (A.Pinner B 34, 4239/40 (1901)).

Als Beispiele seien genannt :
2-α-Pyridyl-2-aminoacrylsäuremethylester, 2-α-Pyridyl-2-methylaminoacrylsäureäthylester, 2-β-Pyridyl-2-amino-acrylsäureäthylester, 2-β-Pyridyl-2-äthylamino-acrylsäureisopropylester, 2-γ-pyridyl-2-aminoacrylsäuremethylester, 2-γ-Pyridyl-2-aminoacrylsäureäthylester, 2-γ-Pyridyl-2-methylamino-acrylsäurebutylester, 2-γ-Pyridyl-2-propylamino-acrylsäurepropylester.

Die erfindungsgemäß verwendbaren Yliden-β-ketocarbonsäureester der Formel V sind teilweise bekannt oder können nach allgemein bekannten Methoden hergestellt werden (Org.Reactions XI, 204 ff. (1967)).

Als Beispiele seien genannt :

Benzylidenacetessigsäuremethylester, 2'-Nitrobenzyliden-acetessigsäuremethylester, 3'-Nitrobenzylidenactessigsäurepropargylester, 3'-Nitrobenzylidenacetessigsäureallylester, 3'-Nitrobenzylidenacetessigsäure-β-äthoxyäthylester, 4'-Nitrobenzylidenacetessigsäureisopropylester, 3'-Nitro-6'-chlorbenzylidenacetessigsäuremethylester, 2'-Cyanbenzylidenpropionylessigsäureäthylester, 3'-Cyanbenzylidenacetessigsäuremethyl-ester, 3'-Nitro-4'-chlorbenzylidenacetessigsäure-tert.-butylester, 2'-Nitro-4'-methoxybenzylidenacetessigsäuremethylester, 2'-Cyan-4'-methylbenzylidenacetessigsäureäthylester, 2'-Azidobenzylidenacetessigsäureäthylester, 2'-Methylmercaptobenzylidenacetessigsäureisopropylester, 2'-Sulfinylmethylbenzylidenacetessigsäureäthylester, 2-Sulfonylmethylacetessigsäureäthylester, (2'-Aethoxy-1'-naphthyliden)-Acetessigsäuremethylester, (1'-Isochinolyl)-Methylenacetessigsäuremethylester, α-Pyridylmethylidenacetessigsäuremethylester, α-Pyridylmethylidenacetessigsäureallylester, α-Pyridylmethylidenacetessigsäurecyclohexylester, β-Pyridylmethylidenacetessigsäure-β-methoxyäthylester, 6-Methyl-α-pyridylmethylidenacetessigsäureäthylester, 4',6'-Dimethoxy-(5'-pyrimidyl)-methylidenacetessigsäureäthylester, (2'-Thenyl)-methylidenacetessigsäureäthylester, (2'-Furyl)-methylidenacetessigsäureallylester, (2'-Pyrryl)-methylidenacetessigsäuremethylester, α-Pyridylmethylidenpropionylessigsäuremethylester,



BAD ORIGINAL

2'-, 3'- oder 4'-Methoxybenzylidenacetessigsäureäthylester, 2'-Methoxybenzylidenacetessigsäurepropargylester, 2'-Isopropoxybenzylidenacetessigsäureäthylester, 3'-Butoxybenzylidenacetessigsäuremethylester, 3',4',5'-Trimethoxybenzylidenacetessigsäureallylester, 2'-Methyl-benzylidenpropionylessigsäuremethylester, 2'-Methylbenzylidenacetessigsäure-β-propoxyäthylester, 3',4'-Dimethoxy-5'-brombenzylidenacetessigsäureäthylester, 2'-, 3'- oder 4'-Chlor/Brom/Fluor/-Jodbenzylidenacetessigsäureäthylester, 3'-Chlorbenzylidenpropionylessigsäureäthylester, 2'-, 3'- oder 4'-Trifluormethylbenzylidenacetessigsäurepropylester, 2'-Carbäthoxybenzylidenacetessigsäureäthylester, 4-Carboxyisopropylbenzylidenacetessigsäureisopropylester.

Benzylidenγ-methoxyacetessigsäureäthylester, 2'-Nitrobenzyliden-γ-methoxyacetessigsäureäthylester, 3'-Methoxybenzyliden-γ-äthoxyacetessigsäuremethylester, 3'-4'-Dimethoxybenzyliden-γ-propoxyacetessigsäurepropylester, 2'-Trifluormethylbenzylidenγ-methoxyacetessigsäureäthylester, 2-Chlorbenzyliden-γ-äthoxyacetessigsäurepropylester, 3-Mercaptobenzyliden--butoxyacetessigsäureäthylester, α-Pyridylmethyliden-γ-isopropoxyacetessigsäure-isopropylester, α-Furylmethyliden-γ-äthoxyacetessigsäure-(β-äthoxyäthyl)-ester.

Die erfindungsgemäß verwendbaren β-Ketocarbonsäureester der Formel VI sowie die Enamino-β-ketocarbonsäureester der Formel VII sind bekannt oder können nach allgemein bekannten Verfahren hergestellt werden (B.Johnson und H.Chesnoff, J.A.C.S. 36, 1744 (1914); Pohl, Schmidt, US-Patentschrift 2 351 366; A. C. Cope, J.A.C.S. 67, 1017 (1945) ).



BAD ORIGINAL

Als Beispiele seien genannt :

β-Dicarbonyl-Verbindungen :

γ-Methoxyacetessigsäuremethylester, γ-Methoxyacetessigsäure-proplester, γ-Aethoxyacetessigsäurepropylester, γ-Propoxyacetessigsäureäthylester, γ-Propoxyacetessigsäureisopropylester, γ-Isopropoxyacetessigsäurebutylester, γ-Butoxyacetessigsäureäthylester, γ-Butoxyacetessigsäureisobutylester, γ-Isobutoxyacetessigsäurepropylester, γ-Methoxypropionylessigsäuremethylester, Formylessigsäureäthylester, Formylessigsäurebutylester, Acetessigsäuremethylester, Acetessigsäureäthylester, Acetessigsäuremethylester, Acetessigsäureisopropylester, Acetessigsäurebutylester, Acetessigsäure-tert.-butylester, Acetessigsäure-(α-oder β-)-hydroxyäthylester, Acetessigsäure-(α- oder β-)methoxyäthylester, Acetessigsäure-(α- oder β-)-äthoxyäthylester, Acetessigsäure-(α- oder β-)-n-propoxyäthylester, Acetessigsäureallylester, Acetessigsäurepropargylester, Propionylessigsäureäthylester, Butyrylessigsäureäthylester, Isobutyrylessigsäureäthylester, Oxalessigsäuredimethylester, Oxalessigsäurediäthylester. Cyclohexandion-1,3.

Enamincarbonsäureester :

γ-Methoxy-β-aminocrotonsäuremethylester, γ-Methoxy-β-aminocrotonsäureäthylester, γ-Aethoxy-β-aminocrotonsäure-propylester, γ-Isopropoxy-β-methylaminocrotonsäureäthylester, γ-Butoxy-γ-methyl-β-aminocrotonsäureäthylester.

β-Aminocrotonsäuremethylester, β-Aminocrotonsäureäthylester, β-Aminocrotonsäureisopropylester, β-Aminocrotonsäurebutylester, β-Aminocrotonsäure-(α- oder β-)-methoxyäthylester,



BAD ORIGINAL

β-Aminocrotonsäure-β-äthoxyäthylester, β-Aminocrotonsäure-β-propoxyäthylester, β-Aminocrotonsäure-t-butyl-ester, β-Aminocrotonsäurecyclohexylester, β-Amino-β-äthyl-acrylsäureäthylester, Iminobernsteinsäuredimethylester, Iminobernsteinsäurediäthylester, Iminobernsteinsäuredipropylester, Iminobernsteinsäuredibutylester, β-Aminoglutarsäuredimethylester, β-Iminoglutarsäurediäthylester, β-Iminoadipinsäuredimethylester, β-Aminoadipinsäurediisopropylester, β-Methylaminocrotonsäuremethylester, β-Aethylaminocrotonsäureäthylester, β-Methyliminoglutarsäurediäthylester, 1,3-dimethyl-2,4-dioxo-6-amino-tetrahydropyrimidin, 1,3-Dipropylamino-2,4-dioxo-6-amino-tetrahydropyrimidin. Die erfindungsgemäß verwendbaren Yliden-β-ketocarbonsäureester der Formel VIII sind noch nicht bekannt. Sie können, wie für Verbindungen der Formel V bereits erwähnt, nach allgemein bekannten Methoden hergestellt werden.

Als Beispiele seien genannt :

α-Pyridylmethyliden-α-pyridoylessigsäuremethylester, β-Pyridylmethyliden-β-pyridoylessigsäureäthylester, Benzylididen-β-pyridoylessigsäure-propylester, 2-Nitrobenzyliden-γ-pyridoylessigsäureisopropylester, 3-Nitrobenzyliden-β-pyridoylessigsäurebutylester, 2-Trifluormethylbenzyliden-α-pyridoylessigsäureisobutylester, 3-Methoxybenzyliden-β-pyridoylessigsäurepropylester, 2-Chlorbenzyliden-α-pyridoylessigsäuremethylester, 2-Methylsulfonylbenzyliden-γ-pyridoylessigsäureäthylester, α-Furylmethyliden-β-pyridoylessigsäuremethylester, α-4-nitrofurylmethyliden-α-pyridoyl-essigsäureäthylester, 4-Jodbenzyliden-γ-pyridolyl-essigsäureäthylester.

Die erfindungsgemäß verwendbaren Aldehyde der Formel IX sind bereits bekannt oder können nach bekannten Methoden hergestellt werden (E. Mosettig, Org. Reactions, VIII, 218 ff, (1954) ).

Als Beispiele seien genannt:
Aldehyde:
Benzaldehyd, 2-, 3- oder 4-Methoxybenzaldehyd, 2-Isopropoxybenzaldehyd, 3-Butoxybenzaldehyd, 3,4-Dioxymethylenbenzaldehyd, 3,4,5-Trimethoxybenzaldehyd, 2-, 3- oder 4-Chlor/Brom/Jod/Fluorbenzaldehyd, 2,4- oder 2,6-Dichlorbenzaldehyd, 2,4-Dimethylbenzaldehyd, 3,5-Diisopropyl-4-methoxybenzaldehyd, 2-, 3- oder 4-Nitrobenzaldehyd, 2,4- oder 2,6-Dinitrobenzaldehyd, 2-Nitro-6-brombenzaldehyd, 2-Nitro-3-methoxy-6-chlorbenzaldehyd, 2-Nitro-4-chlorbenzaldehyd, 2-Nitro-4-methoxybenzaldehyd, 2-, 3- oder 4-Trifluormethylbenzaldehyd, 2-, 3- oder 4-Dimethylaminobenzaldehyd, 4-Dibutylaminobenzaldehyd, 4-Acetaminobenzaldehyd, 2-, 3- oder 4-Cyanbenzaldehyd, 2-Nitro-4-cyanbenzaldehyd, 3-Chlor-4-cyanbenzaldehyd, 2-, 3- oder 4-Methylmercaptobenzaldehyd, 2-Methylmercapto-5-nitrobenzaldehyd, 2-Butylmercaptobenzaldehyd, 2-, 3- oder 4-Methylsulfinylbenzaldehyd, 2-, 3- oder 4-Methylsulfonylbenzaldehyd, Benzaldehyd-2-carbonsäureäthylester, Benzaldehyd-3-carbonsäureisopropylester, Benzaldehyd-4-carbonsäurebutylester, 3-Nitrobenzaldehyd-4-carbonsäureäthylester, Zimtaldehyd, Hydrozimtaldehyd, Formylcyclohexan, 1-Formylcyclohexen-3, 1-Formyl-cyclohexin-1,3, 1-Formylcyclopenten-3, α-, β- oder γ-Pyridinaldehyd, 6-Methylpyridin-2-aldehyd, Furan-2-aldehyd, Thiophen-2-aldehyd und Pyrrol-2-aldehyd, N-Methylpyrrol-2-aldehyd, 2-, 3- oder 4-Azidobenzaldehyd, Pyrimidin-4-aldehyd, 5-Nitro-6-methylpyridin-2-aldehyd, 1- oder 2-Naphthaldehyd, 5-Brom-1-naphthaldehyd, Chinolin-2-aldehyd, 7-Methoxy-chinolin-4-aldehyd, Isochinolin-1-aldehyd.

Als Verdünnungsmittel kommen bei den Verfahrensvarianten a) bis d) Wasser und alle inerten organischen Lösungsmittel in Frage. Hierzu gehören vorzugsweise Alkohole, z. B. niedere Alkylalkohole mit vorzugsweise 1 bis 4 Kohlenstoffatomen, wie Äthanol, Methanol, Isopropanol, Äther, z. B. niedere Dialkyläther (vorzugsweise 3 bis 5 Kohlenstoffatome), wie Diäthyläther oder Ringäther wie Tetrahydrofuran, Dioxan, niedere aliphatische Carbonsäuren (vorzugsweise 2 bis 5 Kohlenstoffatome), wie Essigsäure, Propionsäure, niedere Dialkylformamide (vorzugsweise 1 oder 2 Kohlenstoffatome je Alkylgruppe), wie Dimethylformamid, niedere Alkylnitrile (vorzugsweise 2 bis 4 Kohlenstoffatome), wie Acetonitril, Dimethylsulfoxid, flüssigeheteroaromatische Basen wie Pyridin, sowie Gemische dieser Lösungsmittel einschließlich Wasser untereinander.

Die Reaktionstemperaturen können bei den Verfahrensvarianten a) bis d) in einem größeren Bereich variiert werden. Im allgemeinen arbeitet man zwischen etwa 2o und etwa 15o°C, vorzugsweise zwischen 5o und 1oo°C, insbesondere bei der Siedetemperatur des verwendeten Lösungsmittels.

Die Umsetzung kann bei Normaldruck, aber auch bei erhöhtem Druck durchgeführt werden. Im allgemeinen arbeitet man bei Normaldruck.

Bei der Durchführung der erfindungsgemäßen Verfahrensvarianten a) bis d) werden die an der Reaktion beteiligten Ausgangsstoffe vorzugsweise jeweils etwa in molaren Mengen eingesetzt. Das verwendete Amin bzw. dessen Salz wird zweckmäßig im Überschuß von 1 bis 2 Mol zugegeben. Die Molverhältnisse können über einen weiten Bereich variiert werden ohne, daß das Ergebnis nachteilig beeinflußt wird.

Als neue Wirkstoffe seien zusätzlich genannt:

1,6-Dipropyl-2-(ß-pyridyl)-4-(2'-nitro-4'-methoxyphenyl)-1,4-dihydropyridin-3,5-dicarbonsäuredipropylester,
2-γ-Pyridyl-6-äthyl-4-(3'-nitro-4'-chlorphenyl)-1,4-dihydropyridin-3-carbonsäure-(ß-methoxyäthyl)-ester-5-carbonsäurepropargylester,
N-Isopropyl-2-α-pyridyl-6-methyl-4-(2'-cyanophenyl)-1,4-dihydropyridin-3-carbonsäureisopropylester-5-carbonsäureallylester,
N-Isopropyl-2-γ-pyridyl-6-propoxymethyl-4-(2'-chlorphenyl)-1,4-dihydropyridin-3,5-dicarbonsäuredibutylester,
2-ß-Pyridyl-6-isopropyl-4-(5-pyrimidyl)-dihydropyridin-3-carbonsäuremethyl-5-carbonsäure-sec.-butylester.

1,3,8-Dipropyl-2,4-dioxo-5-(2'-trifluormethylphenyl)-7-α-pyridyl-1,2,3,4,5,8-hexahydro-pyrido-[2,3-d]-pyrimidin-6-carbonsäurepropylester,
1,3-Diäthyl-2,4-dioxo-5-(3'-carbäthoxyphenyl)-7-ß-pyridyl-1,2,3,4,5,8-hexahydropyrimidin-6-carbonsäuremethylester,
N-Methyl-2-α-pyridyl-4-(2'-chlorphenyl)-5,6,7,8-tetrahydrochinolin-4-carbonsäureisopropylester.

Die neuen Verbindungen sind als Arzneimittel, insbesondere als gefäß- und kreislaufbeeinflussende Wirkstoffe verwendba

Die erfindungsgemäßen Verbindungen haben ein breites und vielseitiges pharmakologisches Wirkungsspektrum.

Im einzelnen weisen die erfindungsgemäßen Verbindungen folgende Hauptwirkungen auf:



BAD ORIGINAL

1) Die neuen Verbindungen bewirken bei parenteraler, oraler und perlingualer Gabe eine deutliche und langanhaltende Erweiterung der Coronargefäße.
Diese Wirkung auf die Coronargefäße wird durch einen gleichzeitigen Nitrit-ähnlichen herzentlastenden Effekt verstärkt. Sie beeinflussen beziehungsweise verändern den Herstoffwechsel im Sinne einer Energieersparnis.

2) Die neuen Verbindungen senken den Blutdruck von normotonen und hypertonen Tieren und können somit als antihypertensive Mittel verwendet werden.

3) Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

4) Der Tonus der glatten Muskulatur der Gefäße wird unter der Wirkung der Verbindungen stark vermindert. Diese gefäßspasmolytische Wirkung kann im gesamten Gefäßsystem stattfinden oder sich mehr oder weniger isoliert in umschriebenen Gefäßgebieten (wie z. B. dem Zentralnervensystem) manifestieren.

5) Die Verbindungen haben stark muskulär-spasmolytische Wirkungen, die an der glatten Muskulatur des Magens, Darmtraktes, des Urogenitaltraktes und des Respirationssystems deutlich werden.

6) Die Verbindungen beeinflussen den Cholesterin- beziehungsweise Lipidspiegel des Blutes.

Die neuen Verbindungen sind demnach zur Vorbeugung, Besserung oder Heilung von Erkrankungen geeignet, bei denen insbesondere die oben angegebenen Effekte erwünscht sind.

Zur vorliegenden Erfindung gehören pharmazeutische Zubereitungen, die neben nichttoxischen, inerten pharmazeutisch geeigneten Trägerstoffen eine oder mehrere Verbindungen der Formel I und/oder deren Salze enthalten oder die aus einer oder mehreren Verbindungen der Formel I und/oder deren Salzen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin, und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit, und (i) Gleitmittel, z. B. Talkum, Calcium- und Magnesiumstearat und feste Polyäthylenglykole oder Gemische der unter (a) - (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett, und hohere Ester (z. B. $C_{14}$-Alkohol mit $C_{16}$-Fettsäure) oder Gemische dieser Stoffe.

BAD ORIGINAL

Losungen und Emulsionen konnen neben dem oder den Wirkstoffen die üblichen Tragerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen konnen neben dem oder den Wirkstoffen die üblichen Tragerstoffe, wie flüssige Verdünnungsmittel, z.B. Wasser, Äthylalkohol, Propylenglykol, Suspendiermittel, z. B. äthoxylierte Isostearylalkohole, Polyoxyäthylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusatze, z. B. Pfefferminzöl und Eukalyptusöl und Süßmittel z. B. Saccharin enthalten.

Die therapeutisch wirksamen Verbindungen sollen in den oben aufgeführten pharmazeutischen Zubereitungen vorzugsweise in einer Konzentration von etwa o,1 bis 99,5, vorzugsweise von etwa o,5 bis 95 Gewichtsprozent der Gesamtmischung vorhanden sein.



BAD ORIGINAL

Die oben aufgeführten pharmazeutischen Zubereitungen können außer Verbindungen der Formel I und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der Verbindungen der Formel I und/oder deren Salzen sowie von pharmazeutischen Zubereitungen, die eine oder mehrere Verbindungen der Formel I und/oder deren Salze enthalten, in der Human- und Veterinärmedizin zur Verhütung, Besserung und/oder Heilung der oben angeführten Erkrankungen.

Die Wirkstoffe oder die pharmazeutischen Zubereitungen können vorzugsweise oral, parenteral und/oder rectal, vorzugsweise oral und parenteral, insbesondere perlingual und intravenös appliziert werden.

Im allgemeinen hat es sich als vorteilhaft erwiesen, den oder die Wirkstoffe bei parenteraler (intravenöser) Applikation in Mengen von etwa 0,01 bis etwa 50, vorzugsweise von 0,05 bis 10 mg/kg Körpergewicht je 24 STunden und bei oraler Applikation in Mengen von etwa 0,1 bis etwa 200, vorzugsweise 1 bis 50 mg:kg Körpergewicht je 24 Stunden, gegebenenfalls in Form mehrerer Einzelgaben zur Erzielung der gewünschten Ergebnisse zu verabreichen. Eine Einzelgabe enthält den oder die Wirkstoffe, vorzugsweise in Mengen von etwa 0,01 bis etwa 20 , insbesondere 0,1 bis 2 mg/kg Körpergewicht. Es kann jedoch erforderlich sein, von den genannten Dosierungen



BAD ORIGINAL

abzuweichen, und zwar in Abhängigkeit von der Art und dem Korpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Zur vorliegenden Erfindung gehören auch solche Arzneimittel, die neben Verbindungen der Formel I noch weitere Wirkstoffe enthalten. Vorzugsweise seien genannt: Saluretica, Diuretika, andere kreislaufbeeinflussende Wirkstoffe, Phsychotherapeutica und Analgetika.



BAD ORIGINAL

Die Herstellung der neuen Verbindungen wird durch die folgenden Ausführungsbeispiele exemplarisch erläutert:

Beispiel 1

2-(ß-Pyridyl)-6-methyl-4-phenyl-3-carbonsäureäthylester-5-carbonsäuremethylester

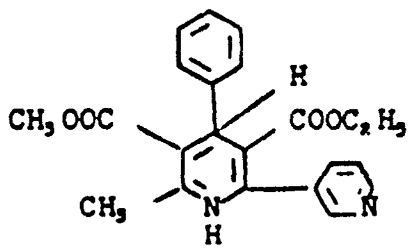

Man erhitzt 10 ccm Benzaldehyd, 19,4 g ß-Pyridoylessigsäureäthylester und 12 g ß-Aminocrotonsäuremethylester in 60 ccm Aethanol über Nacht zum Sieden, kühlt und saugt ab. Gelbe Kristalle vom Fp. 155-156°C,
Ausbeute: 55 % (Variante: d).

Die gleiche Verbindung vom Fp. 156°C wird erhalten durch

12 bis 15 Stunden Erhitzen einer Lösung von 0,1 Mol Benzaldehyd, 0,1 Mol 2-(ß-Pyridyl)-2-aminoacrylsäureäthylester und 0,1 Mol Acetessigsäuremethylester in 80 ccm Methanol,
Ausbeute: 51 % (Variante: c);

Erhitzen von 0,1 Mol Benzyliden-acetessigsäuremethylester und 0,1 Mol 2-(ß-Pyridyl)-2-aminoacrylsäureäthylester 6 Stunden auf 90 bis 100°C oder 12 bis 15 Stunden in 100 ccm Aethanol am Rückfluß,
Ausbeute: 62 % (Variante: b);

Erhitzen von 0,1 Mol Benzyliden-(ß-Pyridoyl)-essigsäureäthylester und 0,1 Mol ß-Aminocrotonsäuremethylester 6 Stunden auf 90 bis 100°C oder 12 bis 15 Stunden in 100 ccm Aethanol am Rückfluß,
Ausbeute: 58 % (Variante: a).

Beispiel 2

2-(γ-Pyridyl)-6-methyl-4-(2'-nitrophenyl)-1,4-dihydropyridin-3-carbonsäureäthylester-5-carbonsäuremethylester.

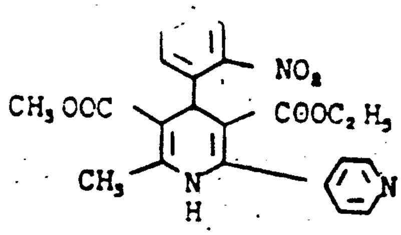

30 g 2-Nitrobenzaldehyd, 24 g ß-Aminocrotonsäuremethylester und 38,8 g γ-Pyridoylessigsäureäthylester werden in 120 ccm Aethanol über Nacht am Rückfluß erhitzt. Nach Kühlung wird abgesaugt und aus Aethanol umkristallisert. Hellgelbe Kristalle vom Fp. 184-186°C,
Ausbeute: 58 % (Variante: d ).

Die gleiche Verbindung vom Fp. 186°C wird erhalten durch

12 bis 15 Stunden Erhitzen einer Lösung von 0,1 Mol 2-Nitrobenzaldehyd, 0,1 Mol 2-(γ-Pyridyl)-2-aminoacrylsäureäthylester und 0,1 Mol Acetessigsäuremethylester in 80 ccm Aethanol,
Ausbeute: 54 % (Variante: c);

Erhitzen von 0,1 Mol 2-Nitrobenzylidenacetessigsäuremethylester und 0,1 Mol 2-($\gamma$-Pyridyl)-2-aminoacrylsäureäthylester 6 Stunden auf 90 bis 100°C oder 12 bis 15 STunden in 100 ccm Aethanol,
Ausbeute: 63 % (Variante: b );

Erhitzen von 0,1 Mol 2-Nitrobenzyliden-($\gamma$-Pyridoyl)-essigsäuremethylester und 0,1 Mol β-Aminocrotonsäuremethylester 6 Stunden auf 90 bis 100°C oder 12 bis 15 STunden in 100 ccm Aethanol am Rückfluß,
Ausbeute: 68 % (Variante: a ).

Beispiel 3

2-($\gamma$-Pyridyl)-6-methyl-4-(3'-nitrophenyl)-1,4-dihydropyridin-3,5-dicarbonsäurediäthylester

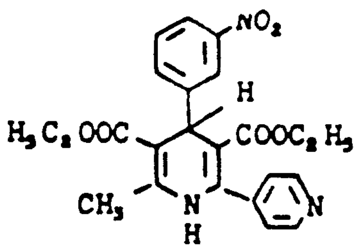

30 g 3-Nitrobenzaldehyd, 26,6 g β-Aminocrotonsäureäthylester und 38,8 g $\gamma$-Pyridoylessigsäureäthylester werden in 120 ccm Aethanol über Nachtam Rückfluß erhitzt. Nach Kühlung wird abgesaugt und aus Aethanol umkristallisiert. Hellgelbe Kristalle vom Fp. 188-190°C,
Ausbeute: 48 % (Variante: d).

Die gleiche Verbindung vom Fp. 190°C wird erhalten durch

12-15 Stdn. Erhitzen einer Lösung von 0,1 mol 3-Nitrobenzaldehyd, 0,1 mol 2-(γ-Pyridyl)-2-aminoacrylsäureäthylester und 0,1 mol Acetessigsäuremethylester in 80 ccm Aethanol
Ausbeute : 45 % (Variante: e);

Erhitzen von 0,1 mol 3-Nitrobenzylidenacetessigsäuremethylester und 0,1 mol 2-(γ-Pyridyl)-2-aminoacrylsäureäthylester
6 Stdn. auf 90-100°C oder
12-15 Stdn. in 100 ccm Aethanol,
Ausbeute: 52 % (Variante: b);

Erhitzen von 0,1 mol 3-Nitrobenzyliden-(γ-Pyridoyl)-essigsäu methylester und 0,1 mol β-Aminocrotonsäuremethylester
6 Stdn. auf 90-100°C oder
12-15 Stdn. in 100 ccm Aethanol am Rückfluß,
Ausbeute: 56 % (Variante: a).

<u>Beispiel 4</u>

2-(γ'-Pyridyl)-6-methyl-4-(2'-trifluormethylphenyl)-1,4-dihydropyridin-3-carbonsäureäthylester-5-carbonsäuremethylester

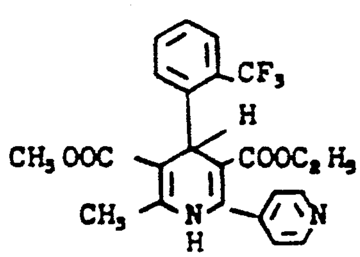

Man erhitzt 17,4 g 2-Trifluormethyl-benzaldehyd, 19,4 γ-Pyric essigsäureäthylester und 12 g β-Aminocrotonsäuremethylester in 60 ccm Aethanol über Nacht zum Sieden, kühlt und saugt ab. Hellgelbe Kristalle vom Fp. 180°C,
Ausbeute: 44 % (Variante: d).

Die gleiche Verbindung vom Fp. 180°C wird erhalten durch

12-15 Stdn. Erhitzen einer Lösung von 0,1 2-Trifluormethyl-benzaldehyd, 0,1 mol 2-(γ-Pyridyl)-2-aminoacrylsäureäthylester und 0,1 mol Acetessigsäuremethylester in 80 ccm Methanol
Ausbeute : 48 % (Variante: c );

Erhitzen von 0,1 mol 2-Trifluormethyl-benzyliden-acetessigsäuremethylester und 0,1 mol 2-(γ-Pyridyl)-2-aminocrotonsäure-äthylester
6 Stdn. auf 90-100°C oder
12-15 Stdn. in 100 ccm Aethanol an Rückfluß
Ausbeute : 58 % (Variante: b );

Erhitzen von 0,1 mol 2-Trifluormethyl-benzyliden-(γ-Pyridoyl)-essigsäureäthylester und 0,1 mol β-Aminocrotonsäuremethylester
6 Stdn. auf 90-100°C oder
12-15 Stdn. in 100 ccm Aethanol am Rückfluß,
Ausbeute: 51 % (Variante: a ).

Beispiel 5

2-(γ-Pyridyl)-6-methyl-4-(3'-trifluormethylphenyl)-1,4-dihydro-pyridin-3,5-dicarbonsäurediäthylester.

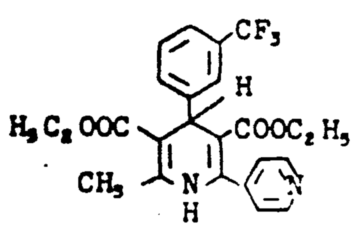

34,8 g 3-Trifluormethylbenzaldehyd, 26,6 g β-Aminocrotonsäureäthylester und 38,8 g γ-Pyridoylessigsäureäthylester werden in 120 ccm Aethanol über Nacht am Rückfluß erhitzt. Nach Kühlung wird abgesaugt und aus Aethanol umkristallisiert. Hellgelbe Kristalle vom Fp. 150-152°C,
Ausbeute: 42 % (Variante: d).
Die gleiche Verbindung vom Fp. 152°C wird erhalten durch

12-15 Stdn. Erhitzen einer Lösung von 0,1 mol 3-Trifluormethylbenzaldehyd, 0,1 mol 2-(γ-Pyridyl)-2-aminoacrylsäureäthylester und 0,1 mol Acetessigsäureäthylester in 80 ccm Aethanol,
Ausbeute :46 % (Variante: c);

Erhitzen von 0,1 mol 3-Trifluormethylbenzylidenacetessigsäureäthylester und 0,1 mol 2-(γ-Pyridyl)-2-aminoacrylsäureäthylester
6 Stdn. auf 90-100°C oder
12-15 Stdn. in 100 ccm Aethanol
Ausbeute :58 % (Variante: b );

Erhitzen von 0,1 mol 3-Trifluormethylbenzyliden-(γ-Pyridoyl)-essigsäureäthylester und 0,1 mol β-Aminocrotonsäuremethylester
6 Stdn. auf 90-100°C oder
12-15 Stdn. in 100 ccm Aethanol am Rückfluß,
Ausbeute: 51 % (Variante: a ).

Beispiel 6

2-(γ-Pyridyl)-6-methyl-4-(4'-nitrophenyl)-1,4-dihydropyridin-3-carbonsäureäthylester-5-carbonsäuremethylester.

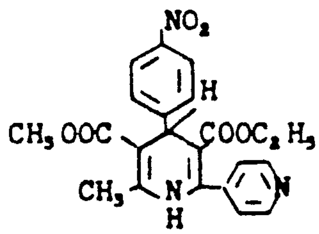

30 g 4-Nitrobenzaldehyd, 24 g β-Aminocrotonsäuremethylester und 38,8 g γ-Pyridoylessigsäureäthylester werden in 120 ccm Aethanol über Nacht am Rückfluß erhitzt. Nach Kühlung wird abgesaugt und aus Aethanol umkristallisiert.
Hellgelbe Kristalle vom Fp. 138-140°C,
Ausbeute: 75 % (Variante: d).
Die gleiche Verbindung vom Fp. 140°C wird erhalten durch

12-15 Stdn. Erhitzen einer Lösung von 0,1 mol 4-Nitrobenzaldehyd, 0,1 mol 2-(γ-Pyridyl)-2-aminoacrylsäureäthylester und 0,1 mol Acetessigsäuremethylester in 80 ccm Aethanol,
Ausbeute : 68 % (Variante: c);

Erhitzen von 0,1 mol 4-Nitrobenzylidenacetessigsäuremethylester und 0,1 mol 2-(γ-Pyridyl)-2-aminoacrylsäureäthylester
6 tdn. auf 90-100°C oder
12-15 Stdn. in 100 ccm Aethanol,
Ausbeute: 76 % (Variante: b);
Erhitzen von 0,1 mol 4-Nitrobenzyliden-(γ-Pyridoyl)-essigsäuremethylester und 0,1 mol β-Aminocrotonsäuremethylester
6 Stdn. auf 90-100°C oder
12-15 Stdn. in 100 ccm Aethanol am Rückfluß,
Ausbeute: 68 % (Variante: a).

Beispiel 7

2-(γ-Pyridyl)-6-methyl-4-(3'-chlorphenyl)-1,4-dihydropyridin-3-carbonsäureäthylester-5-carbonsäuremethylester.

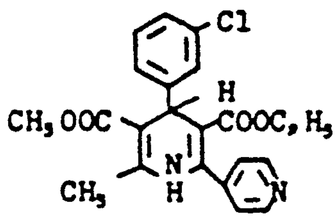

Man erhitzt 14g 3-Chlorbenzaldehyd, 19,4 g γ-Pyridoylessigsäureäthylester und 12 g β-Aminocrotonsäuremethylester in 60 cc Aethanol über Nacht zum Sieden, kühlt und saugt ab.
Gelbe Kristalle vom Fp. 180°C-182°C,
Ausbeute: 62 % (Variante: d).
Die gleiche Verbindung vom Fp. 182°C wird erhalten durch

12-15 Stdn. Erhitzen einer Lösung von 0,1 mol 3-Chlorbenzaldehyd, 0,1 mol 2-(β-Pyridyl)-2-aminoacrylsäureäthylester und 0,1 mol Acetessigsäuremethylester in 80 ccm Methanol,
Ausbeute 65 % (Variante: c);

Erhitzen von 0,1 mol 3-Chlorbenzyliden-acetessigsäuremethyleste und 0,1 mol 2-(β-Pyridyl)-2-aminocrotonsäureäthylester
6 Stdn. auf 90-100°C oder
12-15 Stdn. in 80 ccm Aethanol am Rückfluß,
Ausbeute : 78 % (Variante: b);

Erhitzen von 0,1 mol 3-Chlorbenzyliden(β-Pyridoyl)-essigsäureäthylester und 0,1 mol β-Aminocrotonsäuremethylester
6 Stdn. auf 90-100°C oder
12-15 Stdn. in 80 ccm Aethanol am Rückfluß,
Ausbeute : 72 % (Variante: a).

Beispiel 8

2-(β-Pyridyl)-6-methyl-4-(α-furyl)-1,4-dihydropyridin-3,5-dicarbonsäurediäthylester.

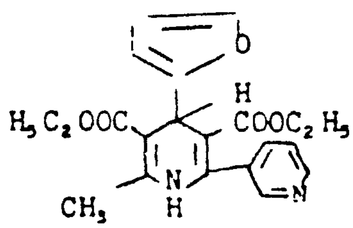

Man erhitzt 8,3 ccm Furfuryl, 19,4 g β-Pyridoylessigsäureäthylester und 12 g β-Aminocrotonsäureäthylester in 60 ccm Aethanol über Nacht zum Sieden, kühlt und saugt ab.
Gelbe Kristalle vom Fp. 165-166°C,
Ausbeute: 52 % (Variante: d).
Die gleiche Verbindung vom Fp. 166°C wird erhalten durch

12-15 Stdn. Erhitzen einer Lösung von 0,1 mol Furfurol, 0,1 mol 2-(β-Pyridyl)-2-aminoacryl-säureäthylester und 0,1 mol Acetessigsäureäthylester in 80 ccm Methanol,
Ausbeute: 56 % (Variante: c):
Erhitzen von 0,1 mol Furfuryliden-acetessigsäureäthylester und 0,1 mol 2-(β-Pyridyl)-2-aminocrotonsäureäthylester
6 Stdn. auf 90-100°C oder
12-15 Stdn. in 100 ccm Aethanol am Rückfluß,
Ausbeute: 75 % (Variante: b);
Erhitzen von 0,1 mol Furfuryliden-(β-Pyridoyl)-essigsäureäthylester und 0,1 mol β-Aminocrotonsäureäthylester
6 Stdn. auf 90-100°C oder
12-15 Stdn. in 100 ccm Aethanol am Rückfluß
Ausbeute :71 % (Variante: a).

Beispiel 9

2,4-Di-(β-pyridyl)-6-methyl-1,4-dihydropyridin-3-carbonsäureäthylester-5-carbonsäuremethylester.

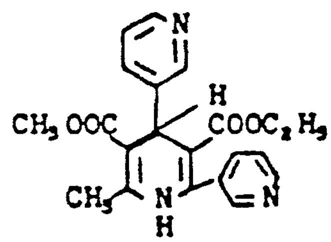

Man erhitzt 10,4 ccam Pyridin-3-aldehyd, 19,4 g β-Pyridoylessigsäureäthylester und 12 g β-Aminocrotonsäuremethylester in 60 ccm Aethanol über Nacht zum Sieden, kühlt und saugt ab. Gelbe Kristalle vom Fp. 162-164°C,
Ausbeute: 75 % (Variante: d).
Die gleiche Verbindung vom Fp. 164°C wird erhalten durch

12-15 Stdn. Erhitzen einer Lösung von 0,1 mol Pyridin-3-aldehyd, 0,1 mol 2-(β-Pyridyl)-2-aminoacrylsäureäthylester un 0,1 mol Acetessigsäuremethylester in 80 ccm Methanol
Ausbeute :69 % (Variante: c);

Erhitzen von 0,1 mol β-Pyridylmethyliden-acetessigsäuremethylester und 0,1 mol 2-(β-Pyridyl)-2-aminocrotonsäureäthylester 6 Stdn. auf 90-100°C oder
12-15 Stdn. in 100 ccm Aethanol am Rückfluß,
Ausbeute: 68 % (Variante: b);
Erhitzen von 0,1 mol β-Pyridylmethyliden-(β-Pyridoyl)-essigsäureäthylester und 0,1 mol β-Aminocrotonsäuremethylester 6 Stdn. auf 90-100°C oder
12-15 Stdn. in 100 ccm Aethanol am Rückfluß,
Ausbeute: 76 % (Variante: a).

Beispiel 10

2,4-Di-(β-pyridyl)-6-methyl-1,4-dihydropyridin-3,5-dicarbonsäure-diäthylester.

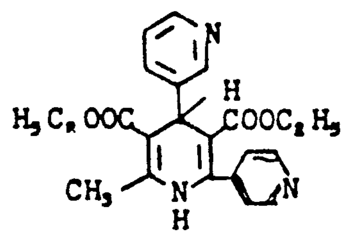

Man erhitzt 10,4 ccm Pyridin-3-aldehyd, 19,4 g β-Pyridoyl-essigsäureäthylester und 12 g β-Aminocrotonsäureäthylester in 60 ccm Aethanol über Nacht zum Sieden, kühlt und saugt ab.

Gelbe Kristalle vom Fp. 155-156°C,

Ausbeute: 72 % (Variante: d).

Die gleiche Verbindung vom Fp. 156°C wird erhalten durch

12-15 Stdn. Erhitzen einer Lösung von 0,1 mol Pyridin-3-aldehyd, 0,1 mol 2-(β-Pyridyl)-2-aminoacrylsäureäthylester und 0,1 mol Acetessigsäureäthylester in 80 ccm Methanol

Ausbeute: 65 % (Variante: c);

Erhitzen von 0,1 mol β-Pyridylmethylidenacetessigsäureäthylester und 0,1 mol 2-(β-Pyridyl)-2-aminocrotonsäureäthylester 6 Stdn. auf 90-100°C oder 12-15 Stdn. in 100 ccm Aethanol am Rückfluß,

Ausbeute: 73 % (Variante: b);

Erhitzen von 0,1 mol β-Pyridylmethyliden-(β-Pyridoyl)-essig-säureäthylester und 0,1 mol β-Aminocrotonsäureäthylester 6 Stdn. auf 90-100°C oder 12-15 Stdn. in 100 ccm Aethanol am Rückfluß,

Ausbeute: 58 % (Variante: a).

**Beispiel 11**

2-(α-Pyridyl)-4-(β-pyridyl)-6-methyl-1,4-dihydropyridin-3-carbonsäureäthylester-5-carbonsäuremethylester.

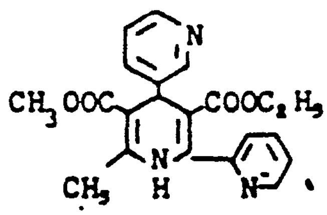

Man erhitz 10,4 ccm Pyridin-3-aldehyd, 19,4 g α-Pyridoyl-essigsäureäthylester und 12 g β-Aminocrotonsäuremethylester in 60 ccm Aethanol über Nacht zum Sieden, kühlt und saugt ab. Gelbbraune Kristalle vom Fp. 138-140°C,
Ausbeute: [illegible] % (Variante: d).

Die gleiche Verbindung vom Fp. 140°C wird erhalten durch

12-15 Stdn. Erhitzen einer Lösung von 0,1 mol Pyridin-3-aldehyd, 0,1 mol 2-(α-Pyridyl)-2-aminoacrylsäureäthylester und 0,1 mol Acetessigsäuremethylester in 80 ccm Methanol,
Ausbeute 72 % (Variante: c);

Erhitzen von 0,1 mol β-Pyridylmethyliden-acetessigsäuremethylester und 0,1 mol 2-(α-Pyridyl)-2-aminocrotonsäureäthylester 6 Stdn. auf 90-100°C oder
12-15 Stdn. in 100 ccm Aethanol am Rückfluß,
Ausbeute: 75 % (Variante: b);

Erhitzen von 0,1 mol β-Pyridylmethyliden-(α-Pyridoyl)-essigsäureäthylester und 0,1 mol β-Aminocrotonsäuremethylester 6 Stdn. auf 90-100°C oder
12-15 Stdn. in 100 ccm Aethanol am Rückfluß,
Ausbeute: 65 % (Variante: a).

Beispiel 12

2-(α-Pyridyl)-4-β-pyridyl-6-methyl-1,4-dihydropyridin-3,5-dicarbonsäure-diäthylester.

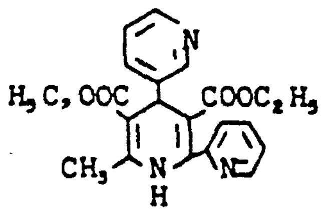

Man erhitzt 10,4 ccm Pyridin-3-aldehyd, 19,4 g α-Pyridoylessigsäureäthylester und 13,3 g β-Aminocrotonsäureäthylester in 60 ccm Aethanol über Nacht zum Sieden, kühlt und saugt ab. Gelbe Kristalle vom Fp. 1[illegible]-130°C,

Ausbeute: 45 % (Variante: d).

Die gleiche Verbindung vom Fp. 130°C wird erhalten durch

12-15 Stdn. Erhitzen einer Lösung von 0,1 mol Pyridin-3-aldehyd, 0,1 mol 2-(α-Pyridyl)-2-aminoacrylsäureäthylester und 0,1 mol Acetessigsäureäthylester in 80 ccm Methanol,

Ausbeute: 40 % (Variante: c);

Erhitzen von 0,1 mol β-Pyridylmethylidenacetessigsäureäthylester und 0,1 mol 2-(α-Pyridyl)-2-aminocrotonsäureäthylester 6 Stdn. auf 90-100°C oder 12-15 Stdn. in 100 ccm Aethanol am Rückfluß,

Ausbeute: 48 %. (Variante: b);

Erhitzen von 0,1 mol β-Pyridylmethyliden-(α-Pyridoyl)-essigsäureäthylester und 0,1 mol β-Aminocrotonsäureäthylester 6 Stdn. auf 90-100°C oder 12-15 Stdn. in 100 ccm Aethanol am Rückfluß,

Ausbeute: 43 % (Variante: a).



BAD ORIGINAL

Beispiel 13

2-(γ-Pyridyl)-6-methyl-4-(3'-trifluormethylphenyl)-1,4-dihydropyridin-3-carbonsäureäthylester-5-carbonsäuremethylester.

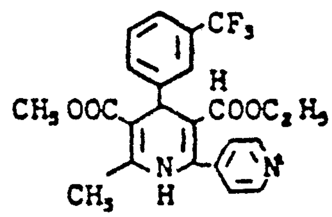

34,8 g 3-Trifluormethylbenzaldehyd, 24 g β-Aminocrotonsäuremethylester und 38,8 g γ-Pyridoylessigsäureäthylester werden in 120 ccm Aethanol über Nacht am Rückfluß erhitzt. Nach Kühlung wird abgesaugt und aus Aethanol umkristallisiert. Hellgelbe Kristalle vom Fp. 184°C,
Ausbeute: 75 % (Variante: d).
Die gleiche Verbindung vom Fp. 184°C wird erhalten durch

12-15 Stdn. Erhitzen einer Lösung von 0,1 mol 3-Fluormethylbenzaldehyd, 0,1 mol 2-(γ-Pyridyl)-2-aminoacrylsäureäthylester und 0,1 mol Acetessigsäuremethylester in 80 ccm Aethanol
Ausbeute : 68 % (Variante: c);

Erhitzen von 0,1 mol 3-Fluormethylidenacetessigsäuremethylester und 0,1 mol 2-(γ-Pyridyl)-2-aminoacrylsäureäthylester 6 Stdn. auf 90-100°C oder
12-15 Stdn. in 100 ccm Aethanol,
Ausbeute: 78 % (Variante: b);
Erhitzen von 0,1 mol 3-Fluormethylbenzyliden-(γ-Pyridoyl)-essigsäureäthylester und 0,1 mol β-Aminocrotonsäuremethylester 6 Stdn. auf 90-100°C oder
12-15 Stdn. 100 ccm Aethanol am Rückfluß,
Ausbeute: 76 % (Variante: a).



BAD ORIGINAL

Beispiel 14

1,6-Dimethyl-2-(γ-pyridyl)-4-(3'-nitrophenyl)-1,4-dihydropyridin 3,5-dicarbonsäurediäthylester.

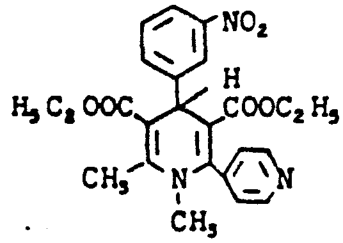

30 g 3-Nitrobenzaldehyd, 29,4 g β-Methylaminocrotonsäureäthylester und 38,8 g γ-Pyridoylessigsäureäthylester werden in 120 c Aethanol über Nacht am Rückfluß erhitzt. Nach Kühlung wird abgesaugt und aus Aethanol umkristallisiert.
Hellgelbe Kristalle vom Fp. 180°C,
Ausbeute: 70 % (Variante: d).
Die gleiche Verbindung vom Fp. 180°C wird erhalten durch

12-15 Stdn. Erhitzen einer Lösung von 0,1 mol 3-Nitrobenzaldehyd, 0,1 mol 2-(γ-Pyridyl)-2-Methylaminoacrylsäureäthylester und 0,1 mol Acetessigsäureäthylester in 80 ccm Aethanol,
Ausbeute : 65 % (Variante: c);

Erhitzen von 0,1 mol 3-Nitrobenzylidenacetessigsäureäthylester und 0,1 mol 2(γ-Pyridyl)-2-Methylaminoacrylsäureäthylester
6 Stdn. auf 90-100°C oder
12-15 Stdn. in 100 ccm Aethanol,
Ausbeute: 74 % (Variante: b);
Erhitzen von 0,1 mol 3-Nitrobenzyliden-(γ-Pyridoyl)-essigsäure methylester und 0,1 mol β-Methylaminocrotonsäureäthylester
6 Stdn. auf 90-100°C oder
12-15 Stdn. in 100 ccm Aethanol am Rückfluß,
Ausbeute: 69 % (Variante: a).

BAD ORIGINAL

Beispiel 15

2-(γ-Pyridyl)-6-methyl-4-(3'-nitrophenyl)-1,4-dihydropyridin-3-carbonsäureäthylester-5-carbonsäuremethylester

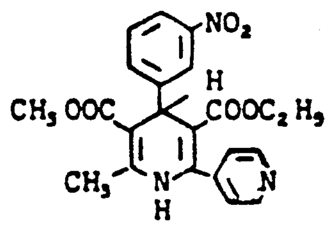

30 g 3-Nitrobenzaldehyd, 24 g β-Aminocrotonsäuremethylester und 38,8 g γ-Pyridoylessigsäureäthylester werden in 120 ccm Aethanol über Nacht am Rückfluß erhitzt. Nach Kühlung wird abgesaugt und aus Aethanol umkristallisiert.
Hellgelbe Kristalle vom Fp. 208-210°C,
Ausbeute: 80 % (Variante: d).
Die gleiche Verbindung vom Fp. 210°C wird erhalten durch

12-15 Stdn. Erhitzen einer Lösung von 0,1 mol 3-Nitrobenzaldehyd, 0,1 mol 2-(γ-Pyridyl)-2-aminoacrylsäureäthylester und 0,1 mol Acetessigsäuremethylester in 80 ccm Aethanol,
Ausbeute : 73 % (Variante: e);

Erhitzen von 0,1 mol 3-Nitrobenzylidenacetessigsäuremethylester und 0,1 mol 2-(γ-Pyridyl)-2-aminoacrylsäureäthylester
6 Stdn. auf 90-100°C oder
12-15 Stdn. in 100 ccm Aethanol,
Ausbeute: 82 % (Variante: b);
Erhitzen von 0,1 mol 3-Nitrobenzyliden-(γ-Pyridoyl)-essigsäuremethylester und 0,1 mol β-Aminocrotnsäuremethylester
6 Stdn. auf 90-100°C oder
12-15 Stdn. in 100 ccm Aethanol am Rückfluß,
Ausbeute: 79 % (Variante: a).

Beispiel 16

2-(β-Pyridyl)-6-methyl-4-(3'-nitrophenyl)-1,4-dihydropyridin-3,5-dicarbonsäurediäthylester

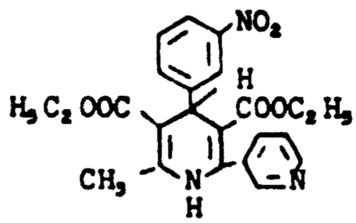

30 g 3-Nitrobenzaldehyd, 26,6 g β-Aminocrotnsäureäthylester und 38,8 g β-Pyridoylessigsäureäthylester werden in 120 ccm Aethanol über Nacht am Rückfluß erhitzt. Nach Kühlung wird abgesaugt und aus Aethanol umkristallisiert.
Hellgelbe Kristalle vom Fp.218-220°C,
Ausbeute: 65 % (Variante: d).
Die gleiche Verbindung vom Fp. 220°C wird erhalten durch

12-15 Stdn. Erhitzen einer Lösung von 0,1 mol 3-Nitrobenzaldehyd, 0,1 mol 2-(β-Pyridyl)-2-aminoacrylsäureäthylester und 0,1 mol Acetessigsäureäthylester in 80 ccm Aethanol,
Ausbeute: 61 % (Variante: c);
Erhitzen von 0,1 mol 3-Nitrobenzylidenacetessigsäureäthylester und 0,1 mol 2-(β-Pyridyl)-2-aminoacrylsäureäthylester
6 Stdn. auf 90-100°C oder
12-15 Stdn. in 100 ccm Aethanol,
Ausbeute: 71 %. (Variante: b);
Erhitzen von 0,1 mol 3-Nitrobenzyliden-(β-Pyridoyl)-essigsäuremethylester und 0,1 mol β-Aminocrotonsäureäthylester
6 Stdn. auf 90-100°C oder
12-15 Stdn. in 100 ccm Aethanol am Rückfluß,
Ausbeute: 76 % (Variante: a).

**Beispiel 17**

2-(γ-Pyridyl)-6-methyl-4-(2'-methoxyphenyl)-1,4-dihydropyridin-3,5-dicarbonsäurediäthylester

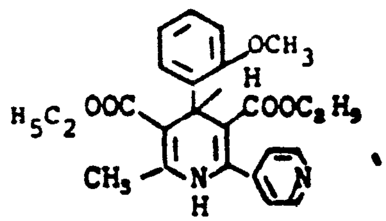

Man erhitzt 13,6 g 2-Methoxy-benzaldehyd, 19,4 g γ-Pyridoyl-essigsäureäthylester und 13,3 g β-Aminocrotonsäureäthylester in 60 ccm Aethanol über Nacht zum Sieden, kühlt und saugt ab.

Gelbe Kristalle vom Fp. 145-146°C,

Ausbeute: 62 % (Variante: d).

Die gleiche Verbindung vom Fp. 146°C wird erhalten durch

12-15 Stdn. Erhitzen einer Lösung von 0,1 mol 2-Methoxy-benzaldehyd, 0,1 mol 2-(γ-Pyridyl)-2-aminoacrylsäureäthylester und 0,1 mol Acetessigsäureäthylester in 80 ccm Methanol

Ausbeute : 57 % (Variante: c);

Erhitzen von 0,1 mol 2-Methoxy-benzyliden-acetessigsäure und 0,1 mol 2-(γ-Pyridyl)-2-aminocrotonsäureäthylester

6 Stdn. auf 90-100°C oder

12-15 Stdn. in 100 ccm Aethanol am Rückfluß,

Ausbeute: 63 % (Variante: b);

Erhitzen von 0,1 mol 2-Methoxy-benzyliden-(γ-Pyridoyl)-essigsäureäthylester und 0,1 mol β-Aminocrotonsäureäthylester

6 Stdn. auf 90-100°C oder

12-15 Stdn. in 100 ccm Aethanol am Rückfluß,

Ausbeute: 65 % (Variante: a).

Beispiel 18

2-(β-Pyridyl)-6-methyl-4-(α-pyridyl)-1,4-dihydropyridin-3,5-dicarbonsäurediäthylester.

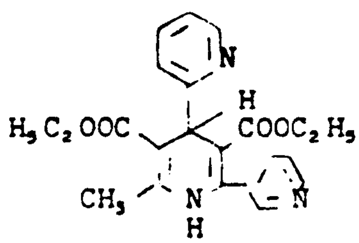

Man erhitzt 10,4 ccm Pyridin-2-aldehyd, 19,4 g β-Pyridoylessigsäureäthylester und 12 g β-Aminocrotonsäureäthylester in 60 ccm Aethanol über Nacht zum Sieden, kühlt saugt ab und fällt aus der Lösung das HCl-Salz.
Aus Aethanol gelbe Kristalle vom Fp. 165-166°C (HCl-Salz)
Ausbeute : 65%. (Variante: d).
Die gleiche Verbindung vom Fp. 166°C wird erhalten durch

12-15 stdg. Erhitzen einer Lösung von 0,1 mol Pyridin-2-aldehyd, 0,1 mol 2-(β-Pyridyl)-2-aminoacrylsäureäthylester und 0,1 mol Acetessigsäureäthylester in 80 ccm Methanol
Ausbeute : 60 % (Variante: c);
Erhitzen von 0,1 mol α-Pyridylmethyliden-acetessigsäureäthylester und 0,1 mol 2-(β-Pyridyl)-2-aminocrotonsäureäthylester
6 Stdn. auf 90-100°C oder
12-15 Stdn. in 100 ccm Aethanol am Rückfluß
Ausbeute : 67 % (Variante: b);
Erhitzen von 0,1 mol α-Pyridylmethyliden-(β-Pyridoyl)-essigsäureäthylester und 0,1 mol β-Aminocrotonsäureäthylester
6 Stdn. auf 90-100°C oder
12-15 Stdn. in 100 ccm Aethanol am Rückfluß
Ausbeute : 69 % (Variante: a).

Beispiel 19

2-(γ-Pyridyl)-6-methyl-4-(2'-chlorphenyl)-1,4-dihydropyridin-3-carbonsäureäthylester-5-carbonsäuremethylester.

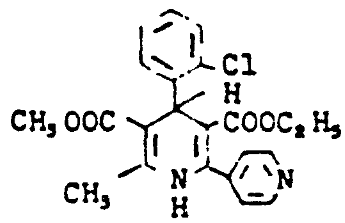

28 g 2-Chlorbenzaldehyd, 24 g β-Aminocrotonsäuremethylester und 38,8 g γ-Pyridoylessigsäureäthylester werden in 120 ccm Äthanol über Nacht am Rückfluß erhitzt. Kühlung wird mit äther. Salzsäure gefällt, abgesaugt und aus Aethanol umkristallisiert. Gelbe Kristalle vom Fp. 224-226°C.
Ausbeute: 85 % (Variante: d).

Die gleiche Verbindung vom Fp. 226°C wird erhalten durch

12-15 stdg. Erhitzen einer Lösung von 0,1 mol 2-Chlorbenzaldehyd, 0,01 mol 2-(γ-Pyridyl)-2-aminoacrylsäureäthylester und 0,1 mol Acetessigsäuremethylester in 80 ccm Aethanol
Ausbeute : 76 % (Variante: c);
Erhitzen von 0,1 mol 2-Chlorbenzylidenacetessigsäuremethylester und 0,1 mol 2-(γ-Pyridyl)-2-aminoacrylsäureäthylester
6 Stdn. auf 90-100°C oder
12-15 Stdn. in 100 ccm Aethanol,
Ausbeute: 86 % (Variante: b);
Erhitzen von 0,1 mol 2-Chlorbenzyliden-(γ-Pyridoyl)-essigsäuremethylester und 0,1 mol β-Aminocrotonsäuremethylester
6 Stdn. auf 90-100°C oder
12-15 Stdn. in 100 ccm Aethanol am Rückfluß,
Ausbeute: 83 % (Variante: a).

Beispiel 20

2-(γ-Pyridyl)-6-äthyl-4-(3'-nitrophenyl)-1,4-dihydropyridin-3,5-dicarbonsäurediäthylester

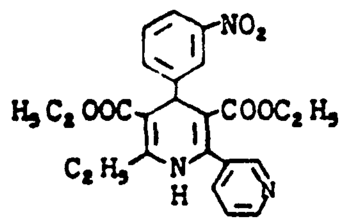

28,8 g 3-Amino-penten-2-säureäthylester ($Kp_{14}$ 105-115°C/ 30 g 3-Nitrobenzaldehyd und 38,8 g γ-Pyridoylessigsäureäthylester werden in 120 ccm Aethanol über Nacht am Rückfluß erhitzt. Nach k(hlung wird mit ätherischer Salzsäure gefällt, abgesaugt und aus Aethanol umkristallisiert. Hellgelbe Kristalle vom Fp. 216°C.

Ausbeute: 65 % (Variante: d).

Die gleiche Verbindung vom Fp. 216°C wird erhalten durch

12-15 stdg. Erhitzen einer Lösung von 0,1 mol 3-Nitrobenzaldehyd, 0,1 mol 2-(γ-Pyridyl)-2-aminocarylsäureäthylester und 0,1 mol Propionylessigsäureäthylester in 80 ccm Aethanol,

Ausbeute: 58 % (Variante: c);

Erhitzen von 0,1 mol 3-Nitrobenzylidenpropionylessigsäureäthylester und 0,1 mol 2-(γ-Pyridyl)-2-aminoacrylsäureäthylester
6 Stdn. auf 90-100°C oder
12-15 Stdn. in 100 ccm Aethanol,

Ausbeute: 67 % (Variante: b);

Erhitzen von 0,1 mol 3-Nitrobenzyliden-(γ-Pyridoyl)-essigsäuremethylester und 0,1 mol 3-Aminopenten-2-säureäthylester
6 Stdn. auf 90-100°C oder
12-15 Stdn. in 100 ccm Aethanol am Rückfluß,

Ausbeute: 71 % (Variante: a).

Beispiel 21

2-(γ-Pyridyl)-6-methyl-4-(3'-nitrophenyl)-1,4-dihydropyridin-3-carbonsäureäthylester-5-carbonsäure-(β-methoxyäthyl)-ester.

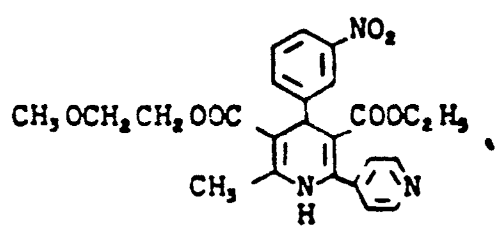

30 g 3-Nitrobenzaldehyd, 32 g β-Aminocrotonsäure-(β-methoxyäthyl)-ester und 38,8 g γ-Pyridoylessigsäureäthylester werden in 120 ccm Aethanol über Nacht am Rückfluß erhitzt. Nach Kühlung wird abgesaugt und aus Aethanol umkristallisiert. Hellgelbe Kristalle vom Fp. 162°C,
Ausbeute: 75 % (Variante: d).
Die gleiche Verbindung vom Fp. 162°C wird erhalten durch 12-15 stdg. Erhitzen einer Lösung von 0,1 mol 3-Nitrobenzaldehyd, 0,1 mol 2-(γ-Pyridyl)-2-aminoacrylsäureäthylester und 0,1 mol Acetessigsäure-(β-methoxyäthyl)-ester in 80 ccm Aethanc
Ausbeute : 69 % (Variante: c);
Erhitzen von 0,1 mol 3-Nitrobenzylidenacetessigsäure-(β-methoxy äthyl)-ester und 0,1 mol 2-(γ-Pyridyl)-2-aminoacrylsäureäthylester
6 Stdn. auf 90-100°C oder
12-15 Stdn. in 100 ccm Aethanol,
Ausbeute: 71 % (Variante: b);
Erhitzen von 0,1 mol 3-Nitrobenzyliden-(γ-Pyridoyl)-essigsäuremethylester und 0,1 mol β-Aminocrotonsäure-β-(methoxyäthyl)-ester
6 Stdn. auf 90-100°C oder
12-15 Stdn. in 100 ccm Aethanol am Rückfluß,
Ausbeute: 78 % (Variante: a).



BAD ORIGINAL

**Beispiel 22**

2-(γ-Pyridyl)-6-methyl-4-(3'-nitrophenyl)-1,4-dihydropyridin-3-carbonsäureäthylester-5-carbonsäureisopropylester.

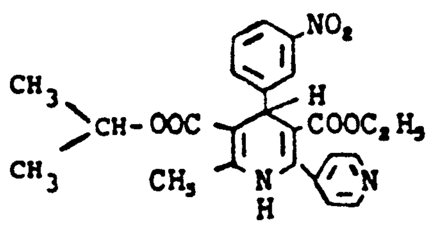

30 g 3-Nitrobenzaldehyd, 28,8 g β-Aminocrotonsäureisopropylester ($Kp_{10}$ 102°C) und 38,8 g β-Pyridoylessigsäureäthylester werden in 120 ccm Aethanol über Nacht am Rückfluß erhitzt. Nach Kühlung wird abgesaugt und aus Aethanol umkristallisiert. Hellgelbe Kristalle vom Fp. 204°C,
Ausbeute: 55 % (Variante: d).
Die gleiche Verbindung vom Fp. 204°C wird erhalten durch 12-15 stdg. Erhitzen einer Lösung von 0,1 mol 3-Nitrobenzaldehyd, 0,1 mol 2-(γ-Pyridyl)-2-aminoacrylsäureäthylester und 0,1 mol Acetessigsäureisopropylester in 80 ccm Aethanol,
Ausbeute : 48 % (Variante: c);
Erhitzen von 0,1 mol 2-Nitrobenzylidenacetessigsäureisopropylester und 0,1 mol 2-(γ-Pyridyl)-2-aminoacrylsäureäthylester
6 Stdn. auf 90-100°C oder
12-15 Stdn. in 100 ccm Aethanol,
Ausbeute: 69 % (Variante: b);
Erhitzen von 0,1 mol 3-Nitrobenzyliden-(γ-Pyridoyl)-essigsäuremethylester und 0,1 mol β-Aminocrotonsäureisopropylester
6 Stdn. auf 90-100°C oder
12-15 Stdn. in 100 ccm Aethanol am Rückfluß,
Ausbeute: 57 % (Variante: a).

Beispiel 23



2-(γ-Pyridyl)-6-methyl-4-(3'-nitrophenyl)-1,4-dihydropyridine-3-carbonsäureäthylester-5-carbonsäure-(β-propoxyäthyl)ester.

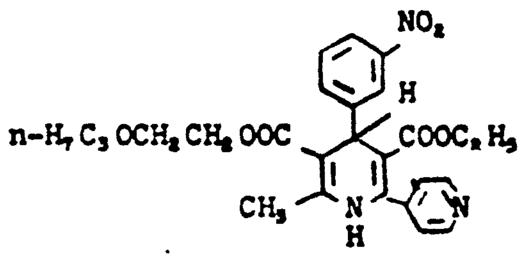

30 g 3-Nitrobenzaldehyd, 38,8 g β-Aminocrotonsäure-(n-propoxyäthyl)-ester ($Kp_{13}$ 150°C) und 38,8 g β-Pyridoylessigsäureäthylester werden in 120 ccm Aethanol über Nacht am Rückfluß erhitzt. Nach Kühlung wird abgesaugt und aus Aethanol umkristallisiert. Hellgelbe Kristalle vom Fp. 134-136°C, Ausbeute: 60 % (Variante: d).

Die gleiche Verbindung vom Fp. 136°C wird erhalten durch

12-15 stdg. Erhitzen einer Lösung von 0,1 mol 3-Nitrobenzaldehyd, 0,1 mol 2-(γ-Pyridyl)-2-aminoacrylsäureäthylester und 0,1 mol Acetessigsäure-(β-n-propoxyäthyl)ester in 80 ccm Aethan
Ausbeute : 53 % (Variante: c);

Erhitzen von 0,1 mol 3-Nitrobenzylidenacetessigsäure-(β-n-propoxyäthyl)-ester und 0,1 mol 2-(γ-Pyridyl)-2-aminoacrylsäureäthylester
6 Stdn. auf 90-100°C oder
12-15 Stdn. in 100 ccm Aethanol,
Ausbeute: 61 % (Variante: b);

Erhitzen von 0,1 mol 3-Nitrobenzyliden-(γ-Pyridoyl)-essigsäure-äthylester und 0,1 mol β-Aminocrotonsäure-(β-n-propoxyäthyl)-este
6 Stdn. auf 90-100°C oder
12-15 Stdn. in 100 ccm Aethanol am Rückfluß,
Ausbeute: 64 % (Variante: a).

Beispiel 24

2-(β-Pyridyl)-6-methyl-4-(3'-nitrophenyl)-1,4-dihydropyridin-3-carbonsäureäthylester-5-carbonsäuremethylester.

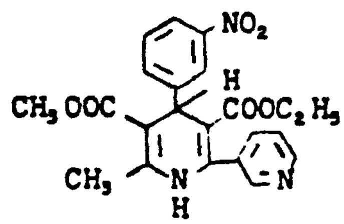

30 g 3-Nitrobenzaldehyd, 24 g β-Aminocrotonsäuremethylester und 38,8 g β-Pyridoylessigsäureäthylester werden in 120 ccm Aethanol über Nacht am Rückfluß erhitzt. Das Umsetzungsprodukt wird heiß abgesaugt und mit Aethanol gewaschen. Hellgelbe Kristalle vom Fp. 224-226°C,

Ausbeute: 74 % (Variante: d).

Die gleiche Verbindung vom Fp. 226°C wird erhalten durch 12-15 stdg. Erhitzen einer Lösung von 0,1 mol 3-Nitrobenzaldehyd, 0,1 mol 2-(β-Pyridyl)-2-aminoacrylsäureäthylester und 0,1 mol Acetessigsäuremethylester in 80 ccm Aethanol,

Ausbeute: 69 % (Variante: c);

Erhitzen von 0,1 mol 3-Nitrobenzylidenacetessigsäuremethylester und 0,1 mol 2-(β-Pyridyl)-2-aminoacrylsäureäthylester 6 Stdn. auf 90-100°C oder 12-15 Stdn. in 100 ccm Aethanol,

Ausbeute: 77 % (Variante: b);

Erhitzen von 0,1 mol 2-Nitrobenzyliden-(β-Pyridoyl)-essigsäuremethylester und 0,1 mol β-Aminocrotonsäuremethylester 6 Stdn. auf 90-100°C oder 12-15 Stdn. in 100 ccm Aethanol am Rückfluß,

Ausbeute: 71 % (Variante: a).



BAD ORIGINAL

**Beispiel 25**

**2-(γ-Pyridyl)-6-methyl-4-(2'-methoxyphenyl)-1,4-dihydropyridin-3-carbonsäureäthylester-5-carbonsäuremethylester**

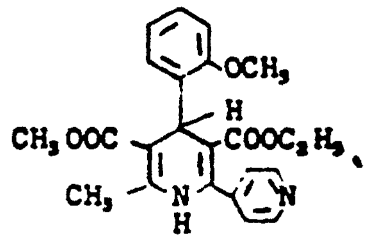

Man erhitzt 13,6 g 2-Methoxybenzaldehyd, 19,4 g γ-Pyridoyl-essigsäureäthylester und 12 g β-Aminocrotonsäuremethylester in 60 ccm Aethanol über Nacht zum Sieden, kühlt und saugt ab. Gelbe Kristalle vom Fp. 84-86°C,

Ausbeute: 45 % (Variante: d).

Die gleiche Verbindung vom Fp. 86°C wird erhalten durch 12-15 stdg. Erhitzen einer Lösung von 0,1 mol 2-Methoxy-benzaldehyd, 0,1 mol 2-(γ-Pyridyl)-2-aminoacrylsäureäthylester und 0,1 Acetessigsäuremethylester in 80 ccm Methanol

Ausbeute : 38 % (Variante: c);

Erhitzen von 0,1 mol 2-Methoxybenzyliden-acetessigsäuremethylester und 0,1 mol 2-(γ-Pyridyl)-2-aminocrotonsäureäthylester 6 Stdn. auf 90-100°C oder 12-15 Stdn. in 100 ccm Aethanol am Rückfluß

Ausbeute : 49 % (Variante: b);

Erhitzen von 0,1 mol 2-Methoxy-benzyliden-(γ-Pyridoyl)-essigsäureäthylester und 0,1 mol β-Aminocrotonsäuremethylester 6 Stdn. auf 90-100°C oder 12-15 Stdn. in 100 ccm Aethanol am Rückfluß,

Ausbeute: 52 % (Variante: a).

BAD ORIGINAL

Beispiel 26

2-(γ-Pyridyl)-6-methyl-4-(3',4',5'-trimethoxyphenyl)-1,4-dihydropyridin-3-carbonsäureäthylester-5-carbonsäuremethylester.

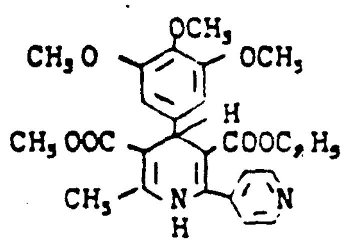

Man erhitzt 19,6 g 3,4,5-Trimethoxy-benzaldehyd, 19,4 g γ-Pyridoylessigsäureäthylester und 12 g β-Aminocrotonsäuremethylester in 60 ccm Aethanol über Nacht zum Sieden, kühlt, saugt ab und fällt mit ätherischer Salzsäure.

Gelbe Krista-le vom Fp. 228°C (HCl-Salz),

Ausbeute: 65 % (Variante: d ).

Die gleiche Verbindung vom Fp. 228°C wird erhalten durch 12-15 stdg. Erhitzen einer Lösung von 0,1 mol 3,4,5-Trimethoxybenzaldehyd, 0,1 mol 2-(γ-Pyridyl)-2-aminoacrylsäureäthylester und 0,1 mol Acetessigsäuremethylester in 80 ccm Methanol

Ausbeute : 57 % (Variante: c );

Erhitzen von 0,1 mol 3,4,5-Trimethoxy-benzyliden-acetessigsäuremethylester und 0,1 mol 2-(γ-Pyridyl)-2-aminocrotnsäureäthylester 6 Stdn. auf 90-100°C oder 12-15 Stdn. in 100 ccm Aethanol am Rückfluß

Ausbeute : 66 % (Variante: b);

Erhitzen von 0,1 mol 3,4,5-Trimethoxy-benzyliden-(γ-Pyridoyl)-essigsäureäthylester und 0,1 mol β-Aminocrotonsäuremethylester 6 Stdn. auf 90-100°C oder 12-15 Stdn. in 100 ccm Aethanol am Rückfluß,

Ausbeute: [illegible] % (Variante: a).



BAD ORIGINAL

Beispiel 27

2-(γ-Pyridyl)-6-methyl-4-(3',4',5'-trimethoxyphenyl)-1,4-dihydro-pyridin-3,5-dicarbonsäurediäthylester

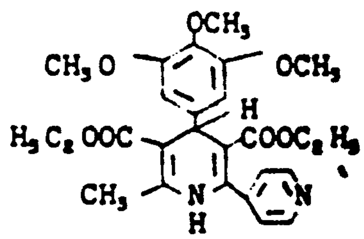

Man erhitzt 19,6 g 3,4,5-Trimethoxybenzaldehyd, 19,4 g β-Pyridoyl-essigsäureäthylester und 13 g β-Aminocrotonsäureäthylester in 60 ccm Aethanol über Nacht zum Sieden, saugt ab und fällt mit äther. Salzsäure. Gelbe Kristalle vom Fp. 205-206°C (HCl-Salz), Ausbeute: 80 % (Variante: d).

Die gleiche Verbindung vom Fp. 206°C wird erhalten durch

12-15 stdg. Erhitzen einer Lösung von 0,1 mol 3,4,5-Trimethoxybenzaldehyd, 0,1 mol 2-(β-Pyridyl)-2-aminoacrylsäureäthylester und 0,1 mol Acetessigsäuremethylester in 80 ccm Methanol
Ausbeute : 71 % (Variante: c);

Erhitzen von 0,1 mol 3,4,5-Trimethoxybenzyliden-acetessigsäuremethylester und 0,1 mol 2-(β-Pyridyl)-2-aminocrotonsäureäthylester
6 Stdn. auf 90-100°C oder
12-15 Stdn. in 100 ccm Aethanol am Rückfluß,
Ausbeute: 76 % (Variante: b);

Erhitzen von 0,1 mol 3,4,5-Trimethoxybenzyliden-(β-Pyridoyl)-essigsäureäthylester und 0,1 mol β-Aminocrotonsäuremethylester
6 Stdn. auf 90-100°C oder
12-15 Stdn. in 100 ccm Aethanol am Rückfluß,
Ausbeute: 69 % (Variante: a).



BAD ORIGINAL

Beispiel 28

2-(γ-Pyridyl)-6-methyl-4-(2'-äthoxycarbonylmethoxy-3-methoxyphenyl)-1,4-dihydropyridin-3-carbonsäureäthylester-5-carbonsäuremethylester

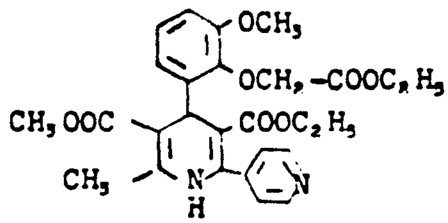

23,8 g 2-Formyl-6-methoxyphenoxyessigsäureäthylester Man erhitzt 19,4 g γ-Pyridoylessigsäureäthylester und 12 g β-Aminocrotonsäuremethylester in 60 ccm Aethanol über Nacht zum Sieden, kühlt und saugt ab.

Ausbeute: 62 % (Variante: d).

Hellgelbe Kristalle vom Fp. 150°C.

Die gleiche Verbindung vom Fp. 150°C wird erhalten durch 12-15 stdg. Erhitzen einer Lösung von 0,1 mol 2-Formyl-6-methoxyphenoxyessigsäureäthylester, 0,1 mol 2-(γ-Pyridyl)-2-aminoacrylsäureäthylester und 0,1 mol Acetessigsäuremethylester in 30 ccm Methanol

Ausbeute : 59 % (Variante: c);

Erhitzen von 0,1 mol 2-Aethoxycarbonylmethoxy-3-methoxybenzyliden-acetessigsäuremethylester und 0,1 mol 2-(γ-Pyridyl)-2-aminocrotonsäureäthylester

6 Stdn. auf 90-100°C oder

12-15 Stdn. in 100 ccm Aethanol am Rückfluß

Ausbeute : 65 % (Variante: b);

Erhitzen von 0,1 mol 2-Aethoxycarbonylmethoxy-3-methoxy-benzyliden-(β-Pyridoyl)-essigsäureäthylester und 0,1 mol γ-Aminocrotonsäuremethylester

BAD ORIGINAL

6 Stdn. auf 90-100°C oder
12-15 Stdn. in 100 ccm Aethanol am Rückfluß
Ausbeute : 71 % (Variante: a).

Beispiel 29

2-(γ-Pyridyl)-6-methyl-(N-methyl-pyrrolyl)-1,4-dihydropyridin-3-carbonsäureäthylester-5-carbonsäureisopropylester

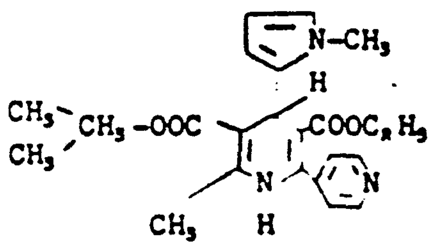

Man erhitzt 11 g N-Methyl-pyrrolaldehyd, 19,4 g γ-Pyridoylessigsäureäthylester und 12 g β-Aminocrotonsäuremethylester in 60 ccm Aethanol über Nacht zum Sieden, saugt ab und fällt mit äther. Salzsäure.
Gelbe Kristalle vom Fp. 166°C (HCl-Salz),
Ausbeute: 40 % (Variante: d).
Die gleiche Verbindung vom Fp. 166°C wird erhalten durch
12-15 Stdn. Erhitzen einer Lösung von 0,1 mol N-Methyl-pyrrolaldehyd, 0,1 mol 2-(γ-Pyridyl)-2-aminoacrylsäureäthylester und 0,1 mol Acetessigsäuremethylester in 80 ccm Methanol
Ausbeute : 32 % (Variante: c);
Erhitzen von 0,1 mol N-Methylpyrrolylmethylidenacetessigsäuremethylester und 0,1 mol 2-(γ-Pyridyl)-2-aminocrotonsäureäthylester
6 Stdn. auf 90-100°C oder
12-15 Stdn. in 100 ccm Aethanol am Rückfluß
Ausbeute : 38 % (Variante: b).

Erhitzen von 0,1 mol N-Methylpyrrolylmethyliden-(γ-Pyridoyl)-essigsäureäthylester und 0,1 mol β-Aminocrotonsäuremethylester
6 Stdn. auf 90-100°C oder
12-15 Stdn. in 100 ccm Aethanol am Rückfluß
Ausbeute : 43 % (Variante: a ).

Beispiel 30

2-(γ-Pyridyl)-6-methyl-4-(3'-fluor-4'-methoxyphenyl)-1,4-dihydropyridin-3-carbonsäureäthylester-5-carbonsäuremethylester

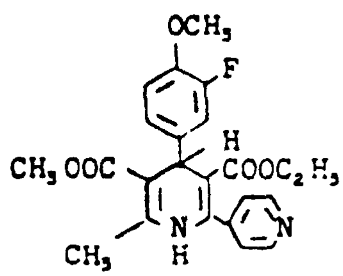

Man erhitzt 15,4 g 3-Fluor-4-methoxybenzaldehyd, 19,4 g γ-Pyridoylessigsäureäthylester und 12 g β-Aminocrotonsäuremethylester in 60 ccm Aethanol über Nacht zum Sieden, kühlt und saugt ab.
Hellgelbe Kristalle vom Fp. 196°C,
Ausbeute: 55 % (Variante: d ).
Die gleiche Verbindung vom Fp. 196°C wird erhalten durch
12-15 Stdn. Erhitzen einer Lösung von 0,1 mol 3-Fluor-4-methoxybenzaldehyd, 0,1 mol 2-(γ-Pyridyl)-2-aminoacrylsäureäthylester und 0,1 mol Acetessigsäuremethylester in 80 ccm Methanol
Ausbeute : 57 % (Variante: c );
Erhitzen von 0,1 mol 3-Fluor-4-methoxybenzyliden-acetessigsäuremethylester und 0,1 mol 2-(γ-Pyridyl)-2-aminocrotonsäureäthylester

6 Stdn. auf 90-100°C oder 12-15 Stdn. in 100 ccm Aethanol am Rückfluß
Ausbeute : 63 % (Variante: b);
Erhitzen von 0,1 mol 3-Fluor-4-methoxybenzyliden-(γ-Pyridoyl)-essigsäureäthylester und 0,1 mol β-Aminocrotonsäuremethylester 6 Stdn. auf 90-100°C oder 12-15 Stdn. in 100 ccm Aethanol am Rückfluß
Ausbeute : 59 % (Variante: a).

Beispiel 31

2-(γ-Pyridyl)-6-methyl-4-(4'-äthylsulfonylphenyl)-1,4-dihydro-pyridin-3,5-dicarbonsäurediäthylester

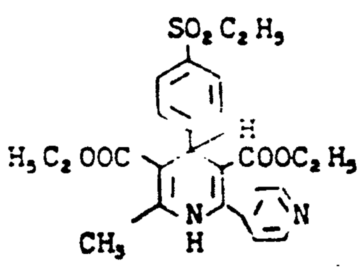

Man erhitzt 19,8 g 4-Aethylsulfonylbenzaldehyd, 19,4 g γ-Pyridoyl-essigsäureäthylester und 13 g β-Aminocrotonsäureäthylester in 60 ccm Aethanol über Nacht zum Sieden, kühlt, fällt mit äther. Salzsäure und saugt ab.
Gelbe Kristalle vom Fp. 220°C,
Ausbeute: 80 % (Variante: d).
Die gleiche Verbindung vom Fp. 220°C wird erhalten durch 12-15 Stdn. Erhitzen einer Lösung von 0,1 mol 4-Aethylsulfonyl, 0,1 mol 2-(γ-Pyridyl)-2-aminoacrylsäureäthylester und 0,1 mol Acetessigsäureäthylester in 80 ccm Methanol
Ausbeute : 82 % (Variante: c);



BAD ORIGINAL

Erhitzen von 0,1 mol 4-Aethylsulfonylbenzyliden-acetessigsäureäthylester und 0,1 mol 2-(γ-Pyridyl)-2-aminocrotonsäureäthylester
6 Stdn. auf 90-100°C oder
12-15 Stdn. in 100 ccm Aethanol am Rückfluß
Ausbeute : 78 % (Variante: b);
Erhitzen von 0,1 mol 4-Aethylsulfonylbenzyliden-(β-Pyridoyl)-essigsäureäthylester und 0,1 mol γ-Aminocrotonsäureäthylester
6 Stdn. auf 90-100°C oder
12-15 Stdn. in 100 ccm Aethanol am Rückfluß,
Ausbeute: 80 % (Variante: a).

Beispiel 32

2-(γ-Pyridyl)-6-methyl-4-(4'-methylmercaptophenyl)-1,4-dihydropyridin-3,5-dicarbonsäurediäthylester

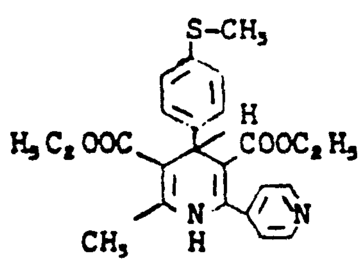

Man erhitzt 15,2 g 4-Methylmercaptobenzaldehyd, 19,4 g γ-Pyridoylessigsäureäthylester und 12 g β-Aminocrotonsäureäthylester in 60 ccm Aethanol über Nacht zum Sieden, kühlt, fällt mit äther. Salzsäure und saugt ab.
Hellgelbe Kristalle vom Fp. 240°C,
Ausbeute: 70 % (Variante: d);

12-15 Stdn. Erhitzen einer Lösung von 0,1 mol 4-Methylmercaptobenzaldehyd, 0,1 mol 2-(γ-Pyridyl)-2-aminoacrylsäureäthylester

und 0,1 mol Acetessigsäureäthylester in 80 ccm Methanol
Ausbeute : 67 % (Variante: c);

Erhitzen von 0,1 mol 4-Methylmercaptobenzyliden-acetessigsäureäthylester und 0,1 mol 2-(γ-Pyridyl)-2-aminocrotonsäureäthylester
6 Stdn. auf 90-100°C oder
12-15 Stdn. in 100 ccm Aethanol am Rückfluß,
Ausbeute: 71 % (Variante: b).

Erhitzen von 0,1 mol 4-Methylmercaptobenzyliden-(γ-Pyridoyl)-essigsäureäthylester und 0,1 mol β-Aminocrotonsäureäthylester
6 Stdn. auf 90-100°C oder
12-15 Stdn. in 100 ccm Aethanol am Rückfluß,
Ausbeute: 73 % (Variante: a).

Beispiel 33

2-(γ-Pyridyl)-6-methyl-4-(2'-methylsulfonylphenyl)-1,4-dihydropyridin-3-carbonsäureäthylester-5-carbonsäuremethylester

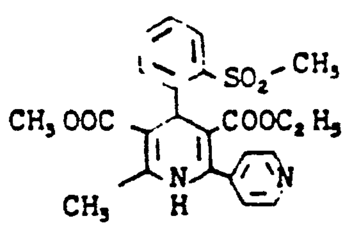

Man erhitzt 18,4 g 2-Methylsulfonylbenzaldehyd, 19,4 g γ-Pyridoylessigsäureäthylester und 12 g β-Aminocrotonsäuremethylester in 60 ccm Aethanol über Nacht zum Sieden, kühlt und saugt ab. Gelbe Kristalle vom Fp. 242°C,
Ausbeute: 55 % (Variante: d).

Die gleiche Verbindung vom Fp. 242°C wird erhalten durch 12-15 Stdn. Erhitzen einer Lösung von 0,1 mol 2-Methylsulfonylbenzaldehyd, 0,1 mol 2-(γ-Pyridyl)-2-aminoacrylsäureäthylester und 0,1 mol Acetessigsäuremethylester in 80 ccm Methanol
Ausbeute : 57 % (Variante: c);

BAD ORIGINAL

Erhitzen von 0,1 mol 2-Methylsulfonylbenzyliden-acetessigsäure-methylester und 0,1 mol 2-(γ-Pyridyl)-2-aminocrotonsäureäthylester
6 Stdn. auf 90-100°C oder
12-15 Stdn. in 100 ccm Aethanol am Rückfluß,
Ausbeute: 59 % (Variante: b);
Erhitzen von 0,1 mol 2-Methylsulfonyl-(γ-Pyridoyl)-essigsäure-äthylester und 0,1 mol ß-Aminocrotonsäuremethylester
6 Stdn. auf 90-100°C oder
12-15 Stdn. in 100 ccm Aethanol am Rückfluß,
Ausbeute: 60 % (Variante: a).

Beispiel 34

2-(γ-Pyridyl)-6-methyl-4-(3'-chlorphenyl)-1,4-dihydropyridin-3-carbonsäureäthylester-5-carbonsäureallylester

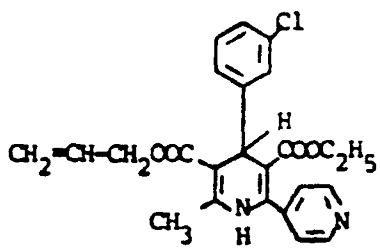

Man erhitzt 15,2 g 3-Chlor-benzaldehyd, 19,4 g γ-Pyridoylessigsäureäthylester und 13 g ß-Aminocrotonsäureallylester in 60 ccm Aethanol über Nacht zum Sieden, kühlt und saugt ab. Gelbe Kristalle vom Fp. 150°C,
Ausbeute: 65 % (Variante: d).

Die gleiche Verbindung vom Fp. 150°C wird erhalten durch 20-25 Stdn. Erhitzen einer Lösung von 0,1 mol 3-Chlorbenzaldehyd, 0,1 mol 2-(γ-Pyridyl)-2-aminoacrylsäureäthylester und 0,1 mol Acetessigsäureallylester in 80 ccm Methanol,
Ausbeute: 58 % (Variante: c);

Erhitzen von 0,1 mol 3-Chlor-benzyliden-acetessigsäureallylester und 0,1 mol 2-(γ-Pyridyl)-2-aminocrotonsäureäthylester 6 Stdn. auf 90-100°C oder 20-25 Stdn. in 100 ccm Aethanol am Rückfluß,
Ausbeute: 67 % (Variante: b);
Erhitzen von 0,1 mol 3-Chlor-benzyliden-(γ-Pyridoyl)-essigsäureäthylester und 0,1 mol ß-Aminocrotonsäureallylester 6 Stdn. auf 90-100°C oder 20-25 Stdn. in 100 ccm Aethanol am Rückfluß,
Ausbeute: 71 % (Variante: a).

Beispiel 35

2,4-(γ-Dipyridyl)-6-methyl-1,4-dihydropyridin-3-carbonsäureäthylester-5-carbonsäuremethylester

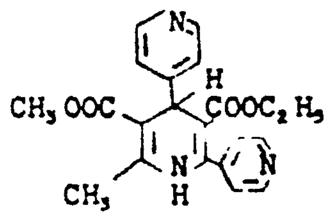

21 ccm Pyridin-4-aldehyd, 24 g β-Aminocrotonsäuremethylester und 38,8 g γ-Pyridoylessigsäureäthylester werden in 120 ccm Aethanol über Nacht am Rückfluß erhitzt. Nach Kühlung wird abgesaugt und aus Aethanol umkristallisiert.
Hellgelbe Kristalle vom Fp. 170°C,
Ausbeute: 50 % (Variante: d).

12-15 stdg. Erhitzen einer Lösung von 0,1 mol Pyridin-4-aldehyd, 0,1 mol 2-(γ-Pyridyl)-2-aminoacrylsäureäthylester und 0,1 mol Acetessigsäuremethylester in 80 ccm Aethanol,
Ausbeute: 45 % (Variante: c);
Erhitzen von 0,1 mol 4-Pyridylmethylidenacetessigsäuremethylester und 0,1 mol 2-( -Pyridyl)-2-aminoacrylsäureäthylester 6 Stdn. auf 90-100°C oder 12-15 Stdn. in 100 ccm Aethanol,
Ausbeute: 51 % (Variante: b);



BAD ORIGINAL

Erhitzen von 0,1 mol 4-Pyridilylmethyliden-(γ-Pyridoyl)-essigsäuremethylester und 0,1 mol β-Aminocrotonsäuremethylester 6 Stdn. auf 90-100°C oder 12-15 Stdn. in 100 ccm Aethanol am Rückfluß, Ausbeute: 53 % (Variante: a).

Beispiel 36

2-(β-Pyridyl)-6-methyl-4-(2'-methylmercaptophenyl)-1,4-dihydropyridin-3-carbonsäureäthylester-5-carbonsäuremethylester

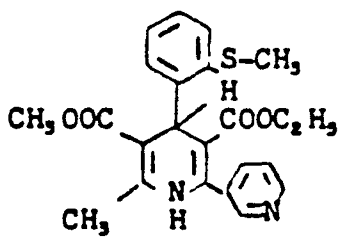

39,6 g 2-Methylmercaptobenzaldehyd, 24 g β-Aminocrotonsäuremethylester und 38,8 g γ-Pyridoylessigsäureäthylester werden in 120 ccm Aethanol über Nacht am Rückfluß erhitzt. Nach Kühlung wird abgesaugt und aus Aethanol umkristallisiert. Hellgelbe Kristalle vom Fp. 144-146°C, Ausbeute: 80 % (Variante: d).

Die gleiche Verbindung vom Fp. 146°C wird erhalten durch 12-15 Stdn. Erhitzen einer Lösung von 0,1 mol 2-Methylmercaptobenzaldehyd, 0,1 mol 2-(γ-Pyridyl)-2-aminoacrylsäureäthylester und 0,1 mol Acetessigsäuremethylester in 80 ccm Aethanol, Ausbeute : 78 % (Variante: c);

Erhitzen von 0,1 mol 2-Methylmercaptobenzylidenacetessigsäuremethylester und 0,1 mol 2-(γ-Pyridyl)-2-aminoacrylsäureäthylester 6 Stdn. auf 90-100°C oder 12-15 Stdn. in 100 ccm Aethanol, Ausbeute: 71 % (Variante: b);

Erhitzen von 0,1 mol 2-Methylmercaptobenzyliden-(γ-Pyridoyl)-essigsäuremethylester und 0,1 mol β-Aminocrotonsäuremethylester
6 Stdn. auf 90-100°C oder
12-15 Stdn. in 100 ccm Aethanol am Rückfluß,
Ausbeute: 76 % (Variante: a).

Beispiel 37

2-(γ-Pyridyl)-6-methyl-4-(2'-methylmercaptophenyl)-1,4-dihydropyridin-3,5-dicarbonsäurediäthylester

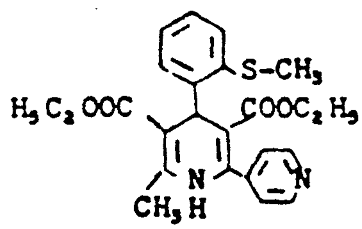

Man erhitzt 19,8 g 2-Methylmercaptobenzaldehyd, 19,4 γ-Pyridoylessigsäureäthylester und 13 g β-Aminocrotonsäureäthylester in 60 ccm Aethanol über Nacht zum Sieden, kühlt und saugt ab. Gelbe Kristalle vom Fp. 148-150°C,
Ausbeute: 55 % (Variante: d).

Die gleiche Verbindung vom Fp. 150°C wird erhalten durch
12-15 Stdn. Erhitzen einer Lösung von 0,1 mol 2-Methylmercaptobenzaldehyd, 0,1 mol 2-(γ-Pyridyl)-2-aminoacrylsäureäthylester und 0,1 mol Acetessigsäureäthylester in 80 ccm Methanol,
Ausbeute : 52 % (Variante: c);

Erhitzen von 0,1 mol 2-Methylmercaptobenzyliden-acetessigsäureäthylester und 0,1 mol 2-(γ-Pyridyl)-2-aminocrotonsäureäthylester
6 Stdn. auf 90-100°C oder
12-15 Stdn. in 100 ccm Aethanol am Rückfluß,
Ausbeute: 57 % (Variante: b);

Erhitzen von 0,1 mol 2-Methylmercaptobenzyliden-(γ-Pyridoyl)-essigsäureäthylester und 0,1 mol β-Aminocrotonsäureäthylester
6 Stdn. auf 90-100°C oder
12-15 Stdn. in 100 ccm Aethanol am Rückfluß,
Ausbeute: 63 % (Variante: a);

**Beispiel 38**

2-(γ-Pyridyl)-6-methyl-4-(2'-nitrophenyl)-1,4-dihydropyridin-3-carbonsäureäthylester-5-carbonsäure-n-propylester

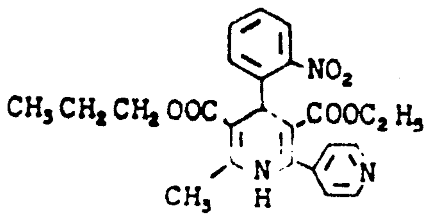

30 g 2-Nitrobenzaldehyd, 28,5 g β-Aminocrotonsäure-n-propylester und 38,8 g β-Pyridoylessigsäureäthylester werden in 120 ccm Aethanol über Nacht [illegible]. Nach Kühlung wird abgesaugt und aus Aethanol umkristallisiert.
Hellgelbe Kristalle vom Fp. 204-206°C,
Ausbeute: 76 % (Variante: d).
Die gleiche Verbindung vom Fp. 206°C wird erhalten durch 12-15 stdg. Erhitzen einer Lösung von 0,1 mol 2-Nitrobenzaldehyd, 0,1 mol 2-(γ-Pyridyl)-2-aminoacrylsäureäthylester und 0,1 mol Acetessigsäure-n-propylester in 80 ccm Aethanol,
Ausbeute: 79 % (Variante: c);
Erhitzen von 0,1 mol 2-Nitrobenzylidenacetessigsäure-n-propylester und 0,1 mol 2-(γ-Pyridyl)-2-aminoacrylsäureäthylester
6 Stdn. auf 90-100°C oder
12-15 Stdn. in 100 ccm Aethanol,
Ausbeute: 68 % (Variante: b);
Erhitzen von 0,1 mol 2-Nitrobenzyliden-(γ-Pyridoyl)-essigsäureäthylester und 0,1 mol β-Aminocrotonsäure-n-propylester
6 Stdn. auf 90-100°C oder
12-15 Stdn. in 100 ccm Aethanol am Rückfluß,
Ausbeute: [illegible] [illegible].



BAD ORIGINAL

Beispiel 39

2-(γ-Pyridyl)-6-methyl-4-(2'-chlorphenyl)-1,4-dihydropyridin-3-carbonsäureäthylester-5-carbonsäure-n-propylester

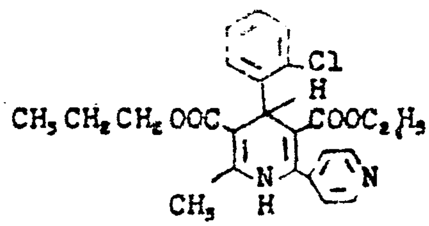

Man erhitzt 14 g 2-Chlorbenzaldehyd, 19,4 g γ-Pyridoylessigsäureäthylester und 14,2 g β-Aminocrotonsäure-n-propylester in 60 ccm Aethanol über Nacht zum Sieden, kühlt und saugt ab.
Gelbe Kristalle vom Fp. 188-190°C,
Ausbeute: 85 % (Variante: d).
Die gleiche Verbindung vom Fp. 190°C wird erhalten durch 12-15 Stdn. Erhitzen einer Lösung von 0,1 mol 2-Chlorbenzaldehyd, 0,1 Mol 2-(γ-Pyridyl)-2-aminoacrylsäureäthylester und 0,1 mol Acetessigsäure-n-propylester in 80 ccm Methanol,
Ausbeute: 87 % (Variante: d;
Erhitzen von 0,1 mol 2-Chlorbenzyliden-acetessigsäure-n-propylester und 0,1 mol 2-(γ-Pyridyl)-2-aminocrotonsäureäthylester 6 Stdn. auf 90-100°C oder
12-15 Stdn. in 100 ccm Aethanol am Rückfluß,
Ausbeute: 79 % (Variante: b);
Erhitzen von 0,1 mol 2-Chlorbenzyliden-(γ-Pyridoyl)-essigsäureäthylester und 0,1 mol β-Aminocrotonsäure-n-propylester 6 Stdn. auf 90-100°C oder
12-15 Stdn. in 100 ccm Aethanol am Rückfluß,
Ausbeute: 78 % (Variante: a).

**Beispiel 40**

2-(γ-Pyridyl)-6-methyl-4-(2'-methoxyphenyl)-1,4-dihydropyridin-3-carbonsäureäthylester-5-carbonsäure-n-propylester

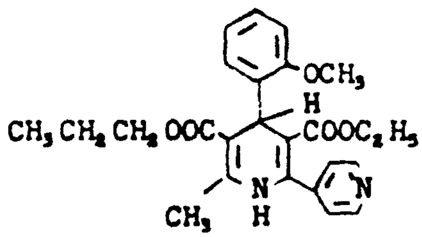

Man erhitzt 13,6 g 2-Methoxybenzaldehyd, 19,4 g γ-Pyridoylessigsäureäthylester und 14,2 g β-Aminocrotonsäure-n-propylester in [illegible] ccm Aethanol über Nacht zum Sieden, kühlt und saugt ab. Gelbe Kristalle vom Fp. 124-126°C,
Ausbeute: 75 % (Variante: d).
Die gleiche Verbindung vom Fp. 126°C wird erhalten durch 12-15 Stdn. Erhitzen einer Lösung von 0,1 mol 2-Methoxybenzaldehyd, 0,1 mol 2-(γ-Pyridyl)-2-aminoacrylsäureäthylester und 0,1 mol Acetessigsäure-n-propylester in 80 ccm Methanol,
Ausbeute: 77 % (Variante: c);
Erhitzen von 0,1 mol 2-Methoxybenzyliden-acetessigsäure-n-propylester und 0,1 mol 2- (γ-Pyridyl)-2-aminocrotonsäureäthylester 6 Stdn. auf 90-100°C oder
12-15 Stdn. in 100 ccm Aethanol am Rückfluß,
Ausbeute: 69 % (Variante: b);
Erhitzen von 0,1 mol 2-Methoxybenzyliden-(γ-Pyridoyl)-essigsäureäthylester und 0,1 mol β-Aminocrotonsäure-n-propylester 6 Stdn. auf 90-100°C oder
12-15 Stdn. in 100 ccm Aethanol am Rückfluß,
Ausbeute: 68 % (Variante: a).

BAD ORIGINAL

Beispiel 41

2-(γ-Pyridyl)-6-methyl-4-(2-nitrophenyl)-1,4-dihydropyridin-3-carbonsäureäthylester-5-carbonsäureallylester

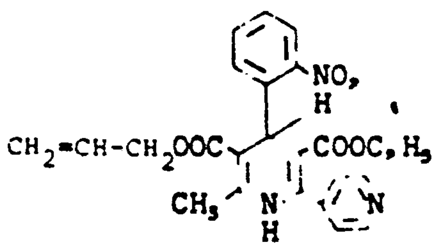

Man erhitzt 15 g 2-Nitro-benzaldehyd, 19,4 g γ-Pyridoyl-essigsäureäthylester und 13 g β-Aminocrotonsäureallylester in 60 ccm Aethanol über Nacht zum Sieden, kühlt und saugt ab.
Gelbe Kristalle vom Fp.173°C,
Ausbeute: 50 % (Variante: d).
Die gleiche Verbindung vom Fp. 173°C wird erhalten durch 20-25 Stdn. Erhitzen einer Lösung von 0,1 mol 2-Nitro-benzaldehyd, 0,1 mol 2-(γ-Pyridyl)-2-aminoacrylsäureäthylester und 0,1 mol Acetessigsäureallylester in 80 ccm Methanol
Ausbeute : 43 % (Variante: c);

Erhitzen von 0,1 mol 2-Nitro-benzyliden-acetessigsäure-allylester und 0,1 mol 2-(γ-Pyridyl)-2-aminocrotonsäure-äthylester
6 Stdn. auf 90-100°C oder
20-25 Stdn. in 100 ccm Aethanol am Rückfluß.
Ausbeute: 52 % (Variante: b);
Erhitzen von 0,1 mol 2-Nitro-benzyliden-(γ-Pyridoyl)-essigsäureäthylester und 0,1 mol β-Aminocrotonsäureallylester

6 Stdn. auf 90-100°C oder
20-25 Stdn. in 100 ccm Aethanol am Rückfluß,
Ausbeute: 55 % (Variante: a).

Beispiel 42

2-(γ-Pyridyl)-6-methyl-4-(3-nitrophenyl)-1,4-dihydropyridin-3-carbonsäureäthylester-5-carbonsäurepropargylester

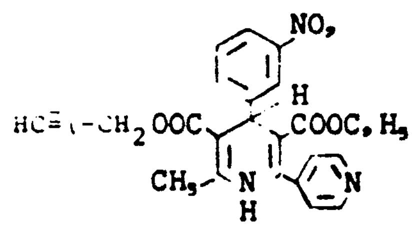

Man erhitzt 15 g 3-Nitrobenzaldehyd, 19,4 g γ-Pyridoyl-essigsäureäthylester und 13 g β-Aminocrotonsäurepropargylester in 60 ccm Aethanol über Nacht zum Sieden, kühlt und saugt ab.
Gelbe Kristalle vom Fp. 208°C,
Ausbeute: 65 % (Variante: d).
Die gleiche Verbindung vom Fp. 208 °C wird erhalten durch 20-25 Stdn. Erhitzen einer Lösung von 0,1 mol 3-Nitro-benzaldehyd, 0,1 mol 2-(γ-Pyridyl)-2-aminoacrylsäureäthylester und 0,1 mol Acetessigsäurepropargylester in 80 ccm Methanol
Ausbeute :58 % (Variante: c);

Erhitzen von 0,1 mol 3-Nitro-benzyliden-acetessigsäure-propargylester und 0,1 mol 2-(γ-Pyridyl)-2-aminocrotonsäure-äthylester
6 Stdn. auf 90-100°C oder

20-25 Stdn. in 100 ccm Aethanol am Rückfluß,
Ausbeute: 61 % (Variante: b);
Erhitzen von 0,1 mol 3-Nitro-benzyliden-(γ-Pyridoyl)-essigsäureäthylester und 0,1 mol ß-Aminocrotonsäure-propargylester 6 Stdn. auf 90-100°C oder
20-25 Stdn. in 100 ccm Aethanol am Rückfluß,
Ausbeute: 67 % (Variante: a).

<u>Beispiel 43</u>

1,3-Dimethyl-2,4-dioxo-5-(3'-nitrophenyl)-7-ß-pyridyl-1,2,3,4,5,8-hexahydro-pyrido/2,3-d/pyrimidin-6-carbonsäureäthylester

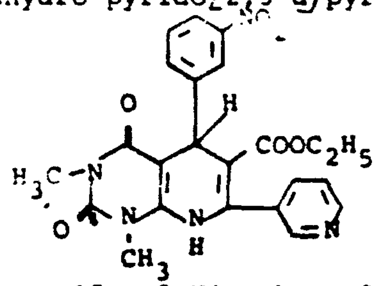

Man erhitzt 15 g 3-Nitrobenzaldehyd, 19,4 g ß-Pyridoylessigsäureäthylester und 15,6 g 1,3-dimethyl-2,4-dioxo-6-amino-tetrahydropyrimidin in 60 ccm Äthanol über Nacht zum Sieden und erhält nach dem Kühlen und Absaugen beige Kristalle vom Fp. 210°C (Äthanol),
Ausbeute: 85 % (Variante: d).
Auf gleiche Weise werden erhalten aus

a) 0,1 mol 2-Nitrobenzaldehyd, 0,1 mol ß-Pyridoylessigsäureäthylester und 0,1 mol 1,3-Dimethyl-2,4-dioxo-6-amino-tetrahydropyrimidin in 80 ccm Äthanol der 1,3-Dimethyl-2,4-dioxo-5-(2'-nitrophenyl)-7-ß-pyridyl-1,2,3,4,5,8-hexahydro-pyrido/2,3-d/pyrimidin-6-carbonsäureäthylester gelbe Kristalle vom Fp. 232°C
Ausbeute: 80 % (Variante: d);

b) 0,1 Mol Pyridin-2-aldehyd, 0,1 mol ß-Pyridoylessigsäureäthylester und 0,1 mol 1,3-Dimethyl-2,4-dioxo-6-amino-tetrahydropyrimidin in 80 ccm Äthanol der 1,3-Dimethyl-2,4-dioxo-5-(α-pyridyl)-7-ß-pyridyl-1,2,3,4,5,8-hexahydro-pyrido[2,3-d]pyrimidin-6-carbonsäureäthylester vom Fp. 200°C (Äthanol, rosarote Kristalle),
Ausbeute: 70 % (Variante: d);

c) 0,1 mol 2-Trifluormethylbenzaldehyd, 0,1 mol ß-Pyridoylessigsäureäthylester und 0,1 mol 1,3-Dimethyl-2,4-dioxo-6-amino-tetrahydropyrimidin in 80 ccm Äthanol der 1,3-Dimethyl-2,4-dioxo-5-(2'-trifluormethylphenyl)-7-ß-pyridyl-1,2,3,4,5,8-hexahydro-pyrido[2,3-d]pyrimidin-6-carbonsäureäthylester, hellgelbe Kristalle vom Fp. 173-174°C (Äthanol),
Ausbeute: 60 % (Variante: d);

d) 0,1 mol 3-Chlorbenzaldehyd, 0,1 mol ß-Pyridoylessigsäureäthylester und 0,1 mol 1,3-Dimethyl-2,4-dioxo-6-amino-tetrahydropyrimidin in 80 ccm Äthanol der 1,3-Dimethyl-2,4-dioxo-5-(3'-chlorphenyl)-7-ß-pyridyl-1,2,3,4,5,8-hexahydro-pyrido-[2,3-d]pyrimidin-6-carbonsäureäthylester, hellbraune Kristalle vom Fp. 222-224°C (Äthanol),
Ausbeute: 55 % (Variante: d).

Beispiel 44

2-(γ-Pyridyl)-6-methyl-4-(3'-nitrophenyl)-1,4-dihydropyridin-3-carbonsäureäthylester-5-carbonsäure-n-butylester

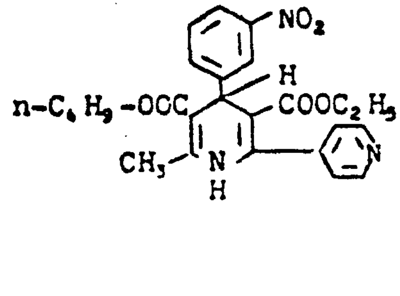

Man erhitzt 15 g 3-Nitrobenzaldehyd, 19,3 g γ-Pyridoylessigsäureäthylester und 15,7 g β-Aminocrotonsäure-n-butylester in 60 ccm Äthanol über Nacht zum Sieden, kühlt und saugt ab. Hellgelbe Kristalle vom Fp. 122-124°C,
Ausbeute: 50 % (Variante: d).

Auf gleiche Weise werden erhalten:

44 a) 2-(β-Pyridyl)-6-methyl-4-(3'-nitrophenyl)-1,4-dihydropyridin-3-carbonsäureäthylester-5-carbonsäure-(β-methoxyäthylester)

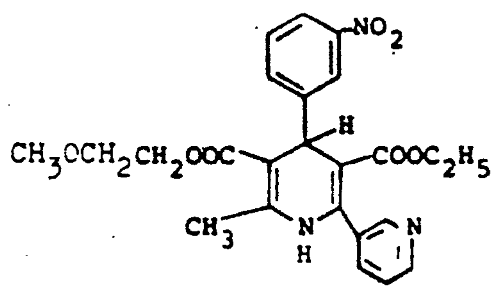

(Variante d)

aus 0,1 mol 3-Nitrobenzaldehyd, 0,1 mol β-Aminocrotonsäure-(β-methoxyäthylester und 0,1 mol β-Pyridoylessigsäureäthylester in 70 ccm Äthanol.
Gelbe Kristalle vom Fp. 162° Ausbeute: 75 % (Variante d)

44 b) 2-(γ-Pyridyl)-6-methyl-4-(2'-nitrophenyl)-1,4-dihydropyridin-3-carbonsäureäthylester 5-carbonsäurebenzylester

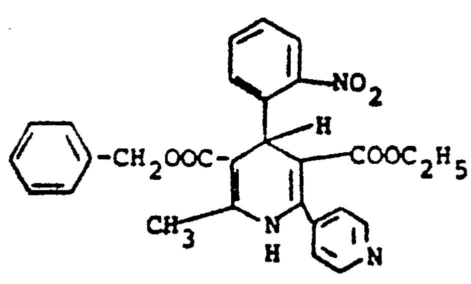

aus 0,1 mol 2-Nitrobenzaldehyd 0,1 mol γ-Pyridoylessigsäureäthylester und 0,1 mol ß-Aminocrotonsäurebenzylester in 80 ccm Äthanol.
Gelbe Kristalle vom Fp. 167-168°C Ausbeute 80 % (Variante d)

44 c) 2-(ß-Pyridyl)-6-methyl-4-(4'-isopropoxyphenyl)-1,4-dihydropyridin-3,5-dicarbonsäurediäthylester

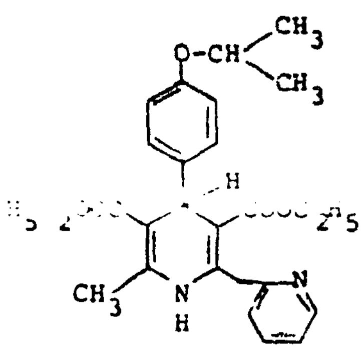

aus 0,1 mol Isopropoxybenzaldehyd 0,1 mol ß-Aminocrotonsäure-äthylester und 0,1 mol ß-Pyridoylessigsäureäthylester in 60 ccm Äthanol.
Hellgelbe Kristalle vom Fp. 128° Ausbeute 55 % (Variante d)

Patentansprüche:

1. 2-Pyridyl-1,4-dihydropyridine der allgemeinen Formel

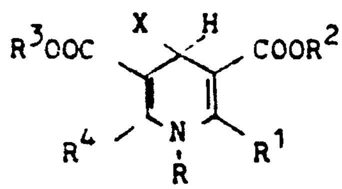

(I)

in welcher

R für Wasserstoff, gegebenenfalls substituiertes Alkyl oder Aralkyl steht

$R^1$ für einen gegebenenfalls substituierten α-, β- oder γ-Pyridyl-Rest steht,

$R^2$ und $R^3$ gleich oder verschieden sind und jeweils für einen gegebenenfalls substituierten Alkyl-, Alkenyl- oder Alkinyl-Rest stehen, welche gegebenenfalls in der Kette durch Heteroatome wie Sauerstoff, Schwefel oder eine -NR'-Gruppe unterbrochen sind, wobei R' Wasserstoff, Alkyl oder Aralkyl bedeutet, wobei die vorgenannten Reste geradkettig, verzweigt oder cyclisch vorliegen,

$R^4$ für Wasserstoff, gegebenenfalls substituiertes Alkyl, Alkoxyalkyl oder Carbalkoxyalkyl steht und

X für gegebenenfalls substituiertes Alkyl oder für einen Arylrest steht, der gegebenenfalls ein, zwei oder drei Substituenten aus der Gruppe Nitro, Cyano, Azido, Halogen, Trifluormethyl, Trifluormethoxy, Phenyl Hydroxy, Amino, Alkyl, Alkoxy, Alkoxycarbonyl, Acyloxy, Acylamino, Monoalkylamino, Dialkylamino und $S(O)_m$-Alkyl trägt, wobei m eine Zahl von 0 bis 2 bedeutet und die vorgenannten Substituenten gleich oder verschieden sind, oder



BAD ORIGINAL

$R^3$ und $R^4$ gemeinsam für einen Alkylen- oder Alkenylenrest stehen, der gegebenenfalls durch Heteroatome wie Sauerstoff, Schwefel oder Stickstoff unterbrochen ist,

sowie ihre pharmakologisch unbedenklichen Salze.

2. 2-Pyridyl-1,4-dihydropyridine der Formel (I) gemäß Anspruch 1, in welcher

R für Wasserstoff, Alkyl mit 1 bis 4 Kohlenstoffatomen, Benzyl oder Phenäthyl steht,

$R^1$ für einen α-, β- oder γ-Pyridyl-Rest steht,

$R^2$ für einen geradkettigen oder verzweigten Alkylrest mit 1 bis 6 Kohlenstoffatomen, insbesondere mit 1 bis 4 Kohlenstoffatomen, der gegebenenfalls durch ein Sauerstoffatom unterbrochen ist steht oder für einen Alkenyl- oder Alkinyl-Rest mit bis zu 4 Kohlenstoffatomen steht,

$R^3$ für einen geradkettigen, verzweigten oder cyclischen Alkyl- oder Alkenyl-Rest, der gegebenenfalls substituiert ist und durch ein Sauerstoffatom in der Kette unterbrochen ist, steht, oder für einen Alkinylrest mit bis zu 4 Kohlenstoffatomen steht,

$R^4$ für Wasserstoff oder Alkyl mit 1 bis 4 Kohlenstoffatomen steht und

X für einen Phenyl- oder Naphthyl-Rest steht, der gegebenenfalls durch 1, 2 oder 3 Substituenten aus der Gruppe Nitro, Halogen, Trifluormethyl, Trifluormethoxy, Cyano, Amino, Monoalkylamino, Dialkylamino, Alkyl, Alkoxy, Alkylmercapto, Alkylsulfinyl, Alkylsulfonyl, Azido und Alkoxycarbonylalkoxy substituiert ist, wobei die genannten Alkyl- und Alkoxyreste



BAD ORIGINAL

vorzugsweise 1 bis 4, insbesondere 1 oder 2 Kohlenstoffatome enthalten,

oder für gegebenenfalls substituiertes Benzyl, Phenathyl, Styryl, Cycloalkyl, Cycloalkenyl mit 5 bis 7 Kohlenstoffatomen, Chinolyl, Isochinolyl, Pyridyl, Pyrimedyl, Furyl, Thienyl oder Pyrylrest steht, wobei als Substituenten vorzugsweise Alkyl, Alkoxy, Dialkylamino, mit jeweils 1 bis 4 Kohlenstoffatomen, insbesondere 1 oder 2 Kohlenstoffatome, Halogen oder Nitro in Frage kommen, sowie ihre pharmakologisch unbedenklichen Salze.

3. Verfahren zur Herstellung von 2-Pyridyl-1,4-dihydropyridinen der Formel (I) dadurch gekennzeichnet, daß man

a) einen ß-Ketocarbonsäureester der Formel

$$R^1-CO-CH_2-COOR^2 \qquad (II)$$

in welcher
$R^1$ und $R^2$ die oben angegebene Bedeutung besitzen,

mit einem Amin oder einem Salz des Amins der Formel

$$H_2N-R \qquad (III)$$

in welcher
R die oben angegebene Bedeutung hat,

gegebenenfalls nach Isolierung des aus (II) und (III) entstehenden Enamins der Formel

$$R^1-\overset{NH-R}{\overset{|}{C}}=CH-COOR^2 \qquad (IV)$$

in welcher
R, $R^1$ und $R^2$ die oben angegebene Bedeutung besitzen,

mit einem Yliden-Derivat der Formel

$$X-CH=\underset{COOR^3}{\underset{|}{C}}-\overset{O}{\overset{\|}{C}}-R^4 \qquad (V)$$

in welcher
X, $R^3$ und $R^4$ die oben angegebene Bedeutung besitzen,

umsetzt,

b) oder 2 Mol ß-Ketocarbonsäureester der Formel

$R^4$-CO-$CH_2$-$COOR^3$ (VI)

in welcher
$R^3$ und $R^4$ die oben angegebene Bedeutung besitzen,

mit einem Amin oder einem Salz des Amins der Formel

$H_2N$-R (III)

in welcher
R die oben angegebene Bedeutung hat,

gegebenenfalls nach Isolierung des aus (IV) und (III) entstehenden Enamins der Formel

$$R^4-\overset{NH-R}{\overset{|}{C}}=CH-COOR^3 \qquad (VII)$$

in welcher
R, $R^3$ und $R^4$ die oben angegebene Bedeutung besitzen,

miteinem Yliden-Derivaten der Formel

$$X-CH=\underset{COOR^2}{\underset{|}{C}}-CO-R^1 \qquad (VIII)$$

in welcher
$R^1$, $R^2$ und X die oben angegebene Bedeutung besitzen,

umsetzt,

c) oder einen ß-Ketocarbonsäureester der Formel

$R^4$-CO-$CH_2$-$COOR^3$ (VI)

in welcher
$R^3$ und $R^4$ die oben angegebene Bedeutung besitzen,

mit einem Enamin der Formel

$$R^1-\overset{NH-R}{\overset{|}{C}}=CH-COOR^2 \qquad (IV)$$

in welcher
R, $R^1$ und $R^2$ die oben angegebene Bedeutung besitzen,

und mit einem Aldehyd der Formel

$$X-CHO \qquad (IX)$$

in welcher
X die oben angegebene Bedeutung hat,

umsetzt,

d) oder einen ß-Ketocarbonsäureester der Formel

$$R^1-CO-CH_2-COOR^2 \qquad (II)$$

in welcher
$R^1$ und $R^2$ die oben angegebene Bedeutung besitzen,

mit einem Enamin der Formel

$$R^4-\overset{NH-R}{\overset{|}{C}}=CH-COOR^3 \qquad (VII)$$

in welcher
R, $R^3$ und $R^4$ die oben angegebene Bedeutung besitzen,

und mit einem Aldehyd der Formel

$$X-CHO \qquad (IX)$$

in welcher
X die oben angegebene Bedeutung hat,

unsetzt und gegebenenfalls aus den gemäß den Verfahrensvarianten a) bis d) erhaltenen Verbindungen in üblicher Weise ein Salz herstellt.

4. Arzneimittel, enthaltend mindestens ein 2-Pyridyl-1,4-dihydropyridin gemäß Anspruch 1.

5. Arzneimittel, enthaltend mindestens ein kreislaufbeeinflussendes 2-Pyridyl-1,4-dihydropyridin gemäß Anspruch 1.

6. Verfahren zur Herstellung von Arzneimitteln, dadurch gekennzeichnet, daß man 2-Pyridyl-1,4-dihydropyridine gemäß Anspruch 1 gegebenenfalls unter Zusatz von inerten, pharmazeutisch unbedenklichen Hilfs- und Trägerstoffen in eine geeignete Applicationsform überführt.

7. Verwendung von 2-Pyridyl-1,4-dihydropyridinen gemäß Anspruch 1 bei der Bekämpfung von Erkrankungen.

8. Verwendung von 2-Pyridyl-1,4-dihydropyridin gemäß Anspruch 1 bei der Bekämfpung von Kreislauferkrankungen.

9. Verfahren zur Behandlung von Kreislauferkrankungen, dadurch gekennzeichnet, daß man 2-Pyridyl-1,4-dihydropyridine gemäß Anspruch 1 Menschen und Tieren im Bedarfsfalle appliziert.



BAD ORIGINAL